# EUROPEAN PATENT APPLICATION

(11) **EP 1 995 309 A1**
(43) Date of publication of application: **26.11.2008**
(21) Application number: 07301058.9
(22) Date of filing: 21.05.2007
(51) Int. Cl.: C12N 5/06, C12N 5/10, C07K 16/00, A61K 39/395, A61K 38/00

(54) **Recombinant protein production in avian EBx® cells**

(71) Applicant: Vivalis, 49450 Roussay (FR)
(72) Inventor: Mehtali, Majid, 44220 Coueron (FR)
(74) Representative: Flesselles, Bruno F.G.

(57) **Abstract**

The invention generally relates to the field of recombinant protein production. More particularly, the invention relates to the use of avian embryonic derived stem cell lines, named EBx®, for the production of proteins and more specifically glycoproteins such as antibodies. The invention is useful for the production of monoclonal IgG1 antibody subtype having high cell-mediated cytotoxic activity. The invention relates to the use of such antibodies as a drug to treat cancers and inflammatory diseases.

## Description

The invention generally relates to the field of recombinant protein production. More particularly, the invention relates to the use of avian embryonic derived stem cell lines, named EBx®, for the production of proteins and more specifically glycoproteins such as antibodies. The invention is useful for the production of monoclonal IgG1 antibody subtype having high cell-mediated cytotoxic activity. The invention relates to the use of such antibodies as a drug to treat cancers and inflammatory diseases.

Glycoproteins mediate many essential functions in human beings including catalysis, signaling, cell-cell communication and molecular recognition and association. Many glycoproteins have been exploited for therapeutic purposes. The oligosaccharide component of protein can affect properties relevant to the efficacy of a therapeutic glycoprotein, including physical stability, resistance to protease attack, interactions with the immune system, pharmacokinetics, and specific biological activity. Such properties may depend not only on the presence or absence, but also on the specific structures, of oligosaccharides. For example, certain oligosaccharide structures mediate rapid clearance of the glycoprotein from the bloodstream through interactions with specific carbohydrate binding proteins, while others can be bound by antibodies and trigger undesired immune reactions (Jenkins et al., Nature Biotechnol., 14:975-81 (1996)). Examples of glycoproteins include erythropoietin (EPO), tissue plasminogen activator (TpA), interferon beta (IFN-b), granulocyte-macrophage colony stimulating factor (GM-CSF), human chorionic gonadotrophin (hCG) and therapeutic monoclonal antibodies (Mabs).

Most antibodies contain carbohydrate structures at conserved positions in the heavy chain constant regions, with each isotype possessing a distinct array of N-linked carbohydrate structures, which variably affect protein assembly, secretion or functional activity (Wright & Morrison (1997) Trends Biotech. 15:26-32). The biological activity of certain G immunoglobulins is dependent on the presence and on the type of glycan structure on the molecule, and in particular on its Fc component. The influence of the presence or the absence of glycan-containing residues on the ability of the antibody to interact with effector molecules (Fc receptors and complement) has been demonstrated. Inhibiting glycosylation of a human IgG1, by culturing in the presence of tunicamycin, causes, for example, a 50-fold decrease in the affinity of this antibody for the FcγRI receptor present on monocytes and macrophages (Leatherbarrow et al. (1985) Molec. Immun., 22:407-415). Binding to the FcγRIII receptor is also affected by the loss of carbohydrates on IgG, since it has been described that a non-glycosylated IgG3 is incapable of inducing lysis of the ADCC type (antibody-dependent cellular cytotoxicity) via the FcγRIII receptor of NK cells (Lund et al. (1990) Molec. Immun. 27:1145-1153). However, beyond the necessary presence of these glycan-containing residues, it is more precisely the heterogeneity of their structure which may result in differences in the ability to initiate effector functions.

IgG molecules of all human and murine subclasses have an N-oligosaccharide attached to the CH2 domain of each heavy chain (at residue Asn 297 for human IgGs). The general structure of N-linked oligosacchararide on IgG is complex type, characterized by a mannosyl-chitobiose core (Man3-GlcNac2-Asn) with or without bisecting GlcNac/L-Fucose (Fuc) and other chain variants including the presence or absence of Gal and sialic acid. In addition, oligosaccharides may contain zero (G0), one (G1) or two (G2) Gal. The structure of the attached N-linked carbohydrate may vary considerably, depending on the degree of processing, and can include high-mannose, multiply-branched as well as biantennary complex oligosaccharides. Typically, there is heterogeneous processing of the core oligosaccharide structures attached at a particular glycosylation site such that even monoclonal antibodies exist as multiple glycoforms. Galactosylation profiles which are variable depending on individuals (human serum IgG1s) have been observed. These differences probably reflect differences in the activity of galactosyltransferases and other enzymes between the cellular clones of these individuals (Jefferis et al. (1990) Biochem J. 268:529-537). Likewise, it has been shown that major differences in antibody glycosylation occur between cell lines, and even minor differences are seen for a given cell line grown under different culture conditions including the composition of the culture medium, the cell density, the pH, the oxygenation (Lifely et al. (1995) Glycobiology 5 (8):813-22; Kumpel et al. (1994) Hum.Antibodies and hybridomas 5:143-151).

Studies have been conducted to investigate the function of oligosaccharide residue on antibody biological activities. Boyd et al (1995 Mol. Immunol. 32:1311-1318) have shown that sialic acid of IgG has no effect on ADCC. Several reports have shown that Gal residues enhance ADCC (kumpel et al. (1994) Hum. Antib. Hybrid 5:143-151; Kumpel et al. (1995) Hum. Antib. Hybrid 6:82-88). Bisecting GlcNac, which is a beta1,4-GlcNac residue transferred to a core beta-mannose (Man) residue, has been implicated in biological residue of therapeutic antibodies (Lifely et al. (1995) Glycobiology 5 (8):813-22). Shield et al. (2002, J. Biol. Chem. 277:26733-26740) have revealed the effect of fucosylated oligosaccharide on antibody effector functions; the Fuc-deficient IgG1 have shown 50-fold increased binding to FcγRIII and enhanced ADCC.

Today, a wide range of recombinant proteins for therapeutic applications (i.e cancer, inflammatory diseases, ...) are composed of glycosylated monoclonal antibodies. For therapeutic and economical reasons, there is a large interest in obtaining higher specific antibody activity. One way to obtain large increases in potency, while maintaining a simple production process in cell line and potentially avoiding significant, undesirable side effects, is to enhance the natural, cell-mediated effector functions of Mabs. Consequently, engineering the oligosaccharides of IgGs may yield optimized ADCC which is considered to be a major function of some of the therapeutic antibodies, although antibodies have multiple therapeutic functions (e.g. antigen binding, induction of apoptosis, and complement-dependent cellular cytotoxicity (CDC)). Company like GLYCART BIOTECHNOLOGY AG (Zurich, CH) has expressed N-acetyl-glucosaminyltransferase III (GnTIII) which catalyzes the addition of the bisecting GlcNac residue to the N-linked oligosaccharide, in a Chinese hamster ovary (CHO) cell line, and showed a greater ADCC of IgG1 antibody produced (WO 99/54342; WO 03/011878; WO 2005/044859). By removing or supplanting fucose from the Fc portion of the antibody, KYOWA HAKKO KOGYO (Tokyo, Japan) has enhanced Fc binding and improve ADCC, and thus the efficacy of the Mab (US6,946,292). More recently, Laboratoire Français du Fractionnement et des Biotechnologies (LFB) (France) showed that the ratio Fuc/Gal in Mab oligosaccharide should be equal or lower than 0.6 to get antibodies with a high ADCC (FR 2 861 080).

However, it still remain a need to have alternative methods for producing human monoclonal antibodies in a cell suitable for large-scale production that has not been engineered to express or to silent appropriate glycosyltransferase, and that provides consistent human-type glycosylation with an enhanced cell-mediated effector functions. This is the goal of the instant invention.

The inventors have now demonstrated that surprisingly monoclonal antibody expressed in avian embryonic derived stem cell line EBx® displays a human-like glycosylation pattern. The inventors also found that a large proportion of IgG1 antibodies population produced in EBx® cells has a common N-linked fucosylated-oligosaccharide structure of a biantennary-type that comprises long chains with terminal GlcNac that are highly galactosylated, and which confer strong ADCC activity to antibodies. The present invention provides avian embryonic derived stem cells EBx®, preferably chicken or duck embryonic derived stem cells EBx®, and more preferably chicken EB 14 cells or duck EB24 cells, genetically engineered to express recombinant proteins, and more specifically antibodies, antibody fragments or fusion proteins which include antibody fragments with increased ADCC activity.

Avian embryonic derived stem cell lines EBx® are cell lines developed by VIVALIS (Nantes, France) who has taken advantage of its expertise in avian biology and in avian embryonic stem (ES) cells to undertake the development of novel stable avian cell lines that fulfill industrial and regulatory specifications. Using a proprietary process (see WO 03/076601), the inventor has generated a series of well characterized and documented cell lines (i.e "*the EBx*® *cells*") with no steps of genetic, chemical or viral immortalization. In a preferred embodiment, the avian cell of the present invention is chicken or duck cells. EBx® cells have been generated using a fully documented 2 steps process, and taking in consideration all regulatory requirements (eg. regular monitoring of the sanitary status of the chicken and duck flocks, use of serum from BSE-free countries, use of pronase instead of trypsin, availability of certificates of origin for all components included in the process, ...).

### Step 1: In vitro culture and expansion of chicken or duck ES cells

Embryonic stem cells are unique in that: (i) they can self-renew indefinitely *in vitro* as undifferentiated cells, (ii) they have unlimited regenerative capacity, (iii) they maintain a stable chromosomal content; (iv) they express high levels of telomerase and specific cell-surface markers. Despite many efforts worldwide, ES cells have been successfully isolated from only a very limited number of species (mouse, human, monkeys). The inventor has dedicated significant resources over the last years to isolate and establish ES cells from different avian species, and mainly from chicken and duck. Such research efforts led to the successful isolation and characterization of chicken ES cells [Pain et al. 1999. Cell Tissues Organs 165: 212-219] and duck ES cells. The inventor then developed proprietary procedures that allow the efficient in vitro culture and large-scale expansion of chicken and duck ES cells without induction of differentiation.

### Step 2: Derivation of EBx® cells:

Then the inventor established a proprietary process to derive stable, adherent or suspension, continuous cell lines from avian ES cells. The process includes the progressive withdrawal of serum, feeder cells and growth factors from the cell culture medium and the adaptation of cells to a suspension culture. These embryonic derived avian cell lines maintained most of the desirable features of ES cells (ie. indefinite proliferation, expression of ES specific markers such as the telomerase, stability of the karyotype) but in addition displayed new "industrial-friendly" characteristics (growth in suspension in serum-free media up to high cell densities).

Based on their attractive biological properties, the inventors selected some chicken EBx® cell lines for further development, such as suspension chicken cell lines EB14 (see WO 03/076601 and WO05/007840) or EBv13. Alternatively, the inventors selected some duck EBx® cell lines for further development, such as EB24, EB26, EB66.

The EBx® cell lines of the invention are "continuous" because they have the characteristics to be cultured *in vitro* over an extended period of time. Advantageously, the cells of the invention are capable of proliferating for at least 50 generation, at least 75 generation, at least 100 generation, at least 125 generation, at least 150 generation, at least 175 generation, at least 200 generation, at least 250 generation. The 250 generation do not constitute a time limit because the cells obtained are still alive and can still be passaged for additional passages. Without to be bond by a theory, it is postulated that the cells of the invention can be cultured "continuously" as long as telomerase is expressed by the cells. Indeed, it is assumed that the high level of telomerase expression of avian cells of the invention is responsible for genetic stability (i.e avian EBx® cells of the invention are diploid) and the continuous cell growth.

By "passage" it is meant the transfer of transplantation of cells, with or without dilution, from one culture vessel to another. It is understood that any time cells are transferred from one vessel to another, a certain portion of the cells may be lost and therefore, dilution of cells, whether deliberate or not, may occur. This term is synonymous with the term 'subculture'. The passage number is the number of times the cells in the culture, that grow either in suspension or in adherence, have been sub-cultured or passed in a new vessel. This term is not synonymous with population doubling or generation which is the time needed by a cell population to replicate one time; that is to say, roughly the time for each cells of a population to replicate. For example, duck or chicken ES have a population doubling time (PDT) of around > 40 hours. The avian EBx® cells of the invention have a PDT of around less than 30 hours, usually less than 24 hours and preferably less than 20 hours. For EBx® cells, there is usually one passage every 3 generations.

By "diploid", it is mean that cells of the invention have two copies (2n) of each chromosome, usually one from the mother and one from the father.

The fact that avian EBx® cell lines of the invention are continuous and diploid (i.e genetically stable) constitutes a remarkable and unique feature because these terms are usually antagonist. Thus, cancer cells and/or immortalized cells obtained by chemical, physical (U.V irradiation, X-ray or g-irradiation, ...) or genetic modification (virus transformation, oncogenes overexpression, ...) are continuous cells because they are able to replicate indefinitely into culture, but they are not genetically stable because they display polyploïd karyotypes. On the other hand, primary cells such as chicken embryonic fibroblasts, MRC5, WI38 which are non-transformed cells, are not continuous because they have a finite life-span after few generation, but they are genetically stable (i.e diploid) cells.

In the present invention, the terms "cell line" and "cells" will be used indistinctly.

The term "avian, "bird", "*aves*" or "*ava*" as used herein is intended to have the same meaning, and will be used indistinctly. "Birds" refer to any species, subspecies or race of organism of the taxonomic class « *ava* ». In a preferred embodiment, "birds" refer to any animal of the taxonomix order:
- "*Anseriformes*" (i.e duck, goose, swan and allies). The order Anseriformes contains about 150 species of birds in three families: the Anhimidae (the screamers), Anseranatidae (the Magpie-goose), and the Anatidae, which includes over 140 species of waterfowl, among them the ducks, geese, and swans. All species in the order are highly adapted for an aquatic existence at the water surface. All are web-footed for efficient swimming (although some have subsequently become mainly terrestrial).
- "*Galliformes*" (i.e chicken, quails, turkey, pheasant and allies). The Galliformes is an order of birds containing the chicken, turkeys, quails and pheasants. About 256 species are found worldwide.
- "*Columbiformes*" (i.e Pigeon and allies). The bird order *Columbiformes* includes the very widespread doves and pigeons.

According to a preferred embodiment, the bird of the invention are selected among the birds that does not comprises avian leucosis virus type E (ALV-E) and endogenous avian virus (EAV) proviral sequences in its genome. A man skilled in the art is able to determine whether ALV-E and EAV sequences are present in a bird genome (Johnson et Heneine, 2001, J. Virol 75:3605-3612; Weissmahr et al., 1997, J. Virol 71:3005-3012). Preferably the bird is selected in the group comprising *Anseriformes* (i.e duck, goose, swan), turkeys, quails, Japanese quail, Guinea fowl, Pea Fowl. Therefore, cells derived from such bird do not produce replication-competent endogenous ALV-E and/or EAV particles. In a preferred embodiment, the bird of the present invention is selected among the group comprising ducks, geese, swans, turkeys, quails and Japanese quails, Guinea Fowls and Pea Fowls. According to a more preferred embodiment, the bird is a duck, more preferably a Pekin or Moscovy ducks. According to a more preferred embodiment, the bird is a Pekin duck. Therefore, the instant invention provides a process for obtaining continuous diploid duck cell lines derived from embryonic stem cells (ES), wherein said duck cell lines do not produce replication-competent endogenous retrovirus particles. Example of duck EBx® cell lines of the invention are EB24, EB26, EB66 or their subclones there of, such as EB24-12.

According to a second preferred embodiment, the bird of the invention are selected among the birds that does not comprises complete ALV-E proviral sequences in its genome but eventually EAV proviral sequences. A man skilled in the art is able to determine whether partial or complete ALV-E and EAV sequences are present in a bird genome (Johnson and Heneine, 2001). Several chicken strains have been selected by breeding that do not contain complete ALV-E proviral sequences (i.e: ev-0 strain) and therefore do not produce infectious ALV-E retroparticles, such as:
- Line 0 domestic chicken of East Lansing USDA poultry stock (ELL-0 strain). The East Lansing Line-0 chickens do not contain any endogenous viral (ev) loci related to ALV (Dunwiddie and Faras, 1985 Proc. Natl. Acad. Sci USA 82:5097-5101).
- Line 22 white leghorn chicken of Charles River (SPAFAS).
- Lines DE and PE11 from Institut National de la Recherche Agronomique (Domaine de Magneraud, Surgères, France).

Therefore, cells derived from ev-0 birds do not produce replication-competent endogenous ALV-E particles. According to a preferred embodiment, the bird is an ev-0 domestic chicken (*Gallus Gallus* subspecies *domesticus),* preferably selected among ELL-0, Line 22, DE and PE11. Usually, ev-0 chickens still contain EAV proviral sequence but so far no infectious EAV isolates have been identified. Therefore, the instant invention provides a process for obtaining continuous diploid chicken cell lines derived from embryonic stem cells (ES) of ev-0 chicken strains, wherein said ev-0 chicken cell lines do not produce replication-competent endogenous retrovirus particles.

According to a third embodiment, the bird of the invention are selected among the birds that comprise complete and/or incomplete ALV-E and EAV proviral sequences in its genome but that are unable to produce replication competent ALV-E and EAV retroparticles. A man skilled in the art is able to determine whether ALV-E and/or EAV infectious and/or non-infectious retroparticles are produced from a bird cells (Johnson and Heneine, 2001; Weissmahr et al., 1996). Preferably the bird is selected in the group comprising specific pathogen free (SPF) chicken, preferably from Valo strain. Example of chicken EBx® cell lines of the invention is EBv13 cell line.

In the instant invention, by the term "endogenous retroviral particle" or "endogenous retrovirus particle", terms that could be used indistinctively, it is meant a retroviral particle or retrovirus encoded by and/or expressed from ALV-E or EAV proviral sequences present in some avian cell genomes. In the birds, ALV-E proviral sequences are known to be present in the genome of domestic chicken (except Line-0 chicken), red jungle fowl and Ringneck Pheasant. In the birds, EAV proviral sequences are known to be present in all genus *gallus* that includes domestic chicken, Line-0 chicken, red jungle fowl, green jungle fowl, grey jungle fowl, Ceylonese jungle fowl and allies) (see Resnick et al., 1990, J. Virol., 64:4640-4653).

By "replication-competent" it is meant that the endogenous retroviral particles are infectious, that is to say that such retroviral particules are able to infect and to replicate in avian cells of the invention.

The process of establishment of continuous diploid avian cell lines, named EBx®, of the invention comprises two steps:
a) isolation, culture and expansion of embryonic stem (ES) cells from birds that do not contain complete endogenous proviral sequences, or a fragment thereof, susceptible to produce replication competent endogenous retroviral particles, more specifically EAV and/or ALV-E proviral sequences or a fragment thereof, in a complete culture medium containing all the factors allowing their growth and in presence of a feeder layer and supplemented with animal serum; optionally, said complete culture medium may comprise additives, such as additional amino-acids (i.e glutamine, ...), sodium pyruvate, beta-mercaptoethanol, protein hydrolyzate of non-animal origin (i.e yeastolate, plant hydrolyzates, ...);
b) passage by modifying the culture medium so as to obtain a total withdrawal of said factors, said feeder layer and said serum, and optionally said additives, and further obtaining adherent or suspension avian cell lines, named EBx®, that do not produce replication-competent endogenous retrovirus particles, capable of proliferating over a long period of time, in a basal medium in the absence of exogenous growth factors, feeder layer and animal serum.

The modification of the culture medium of step b) of the process of establishment EBx® cell lines, so as to obtain progressive or total withdrawal of growth factors, serum and feeder layer, can be made simultaneously, successively or separately. The sequence of the weaning of the culture medium may be chosen among:
- feeder layer / serum / growth factors;
- feeder layer / growth factors / serum;
- serum / growth factors / feeder layer;
- serum / feeder layer / growth factors;
- growth factors / serum / feeder layer;
- growth factors / feeder layer / serum.

In a preferred embodiment, the sequence of the weaning is growth factors / feeder layer / serum.

According to a preferred embodiment, the avian embryonic stem cells according to step
a) of the invention are collected from avian embryo at oviposition, that is to say when the egg is laid. According to Sellier et al. (2006, J. Appl. Poult. Res., 15:219-228), oviposition corresponds to the following development stages according to Eyal-Giladi's classification (EYAL-GILADI's classification: EYAL-GILADI and KOCHAN, 1976, « From cleavage to primitive streack formation : a complementary normal table and a new look at the first stages of the development in the chick ». "General Morphology" Dev. Biol. 49:321-337):
   - Muscovy duck: stage VII
   - Guinea fowl: stage VII - VII I
   - Turkey: stage VII-VIII
   - Pekin duck: stage VIII
   - Chicken: Stage X
   - Japanese Quail: stage XI
   - Goose: stage XI.

Preferably, the duck embryonic stem (ES) cells of step a) is obtained by dissociating embryo(s) at around stage VIII (oviposition) of Eyal-Giladi's classification. Preferably, the chicken embryonic stem (ES) cells, preferably from ev-0 chicken strain, of step a) is obtained by dissociating embryo(s) at around stage X (oviposition) of Eyal-Giladi's classification. Alternatively, the avian embryonic stem cells according to step a) of the invention are collected from embryo before oviposition. The main limitations encountered before oviposition is the fact that the egg has to be surgically removed from hens and that the amount of ES cells per embryo is less important. Moreover at very early stages of avian embryo development, ES cells are not well individualized rendering difficult *in vitro* culture of ES cells. A man skilled in the Art will be able to define the timeframe prior egg laying that allows to collect avian ES cells. Alternatively, the avian embryonic stem cells according to step a) of the invention may be collected from avian embryo after oviposition up to hatching. However, avian embryonic stem cells will progressively enter into differentiation to generate differentiated tissues; therefore, it is preferred to collect avian ES not to long after the lay. A man skilled in the Art will be able to define the timeframe after egg laying that allows to collect avian embryonic stem cells.

These avian embryonic stem cells are characterized by a slow doubling time comprises between 48 to 72 hours in culture at 39°C.

Without to be bound by a theory, the defined cell culture conditions of avian ES cells followed by the progressive weaning in grow factors, feeder layer and serum, allow to adapt and select cells that maintain most of the desirable feature of ES cells (stability of karyotype, indefinite proliferation, expression of ES markers) but in addition display industrial-friendly characteristics like growth in suspension up to high cell densities in serum-free medium. Telomerase constitutes one of the most important ES markers. Due to the sustained and maintained telomerase expression over the cell passages, EBx® cell are continuous (i.e immortal) but in addition are genetically stable (i.e diploid).

The present invention provides a process for obtaining continuous diploid avian cell lines derived from ES cells (named EBx® cell lines or EBx® cells), wherein said avian cell lines do not produce replication competent endogenous retroviral particles, said process comprising the following steps of:
a) isolating bird embryo(s), preferably from duck or from chicken, preferably ev-0 chicken, at a developmental stage comprises from around stage VI of Eyal-Giladi's classification (EYAL-GILADI's classification: EYAL-GILADI and KOCHAN, 1976, «From cleavage to primitive streack formation: a complementary normal table and a new look at the first stages of the development in the chick ». "General Morphology" Dev. Biol., 49:321-337) and before hatching, preferably around oviposition. Preferably, the genome of said bird does not contain endogenous proviral sequences susceptible to produce replication competent endogenous retroviral particles;
b) suspending avian embryonic stem (ES) cells obtained by dissociating embryo(s) of step a) in a basal culture medium supplemented with:
   - Insulin Growth factor 1 (IGF-1) and Ciliary Neurotrophic factor (CNTF);
   - animal serum; and
   - optionally, growth factors selected in the group comprising interleukin 6 (IL-6), interleukin 6 receptor (IL-6R), Stem cell Factor (SCF) and Fibroblast Growth Factor (FGF);
c) seeding the suspension of ES cells obtained in step b) on a layer of feeder cells and further culturing the ES cells for at least one passage;
d) optionally withdrawing all the growth factors selected from the group comprising IL-6, IL-6R, SCF, FGF from the culture medium over a range of several passages from 1 to around 15 passages, and further culturing the avian ES cells for at least one passage. Preferably, the withdrawing of all the growth factors selected from the group comprising IL-6, IL-6R, SCF, FGF from the culture medium is performed simultaneously over one passage. Usually, the withdrawing of IL-6, IL-6R, SCF, FGF is performed at around passage 10 to 15;
e) withdrawing IGF-1 and CNTF from the culture medium and further culturing the avian ES cells for at least one passage. Preferably, the withdrawing of the growth factors selected from the group comprising IGF-1 and CNTF from the culture medium is performed simultaneously, over one passage. Usually, the withdrawing of IGF-1 and CNTF is performed at around passage N° 15 to N° 25. Alternatively, the withdrawing of IGF-1 and CNTF is performed by progressive decreasing over several passages (at least 2 passages and approximately up to 15 passages);
f) progressively decreasing the concentration of feeder cells in the culture medium so as to obtain a total withdrawal of feeder layer after several passages, and further culturing the cells;
g) optionally, progressively decreasing the concentration of additives in the culture medium so as to obtain a total withdrawal of additives after at least one passage; and,
h) optionally, progressively decreasing the concentration of animal serum in the culture medium so as to obtain a total withdrawal of animal serum after several passages; and,
i) obtaining adherent avian cell lines, named EBx®, derived from ES cells capable of proliferating in a basal medium in the absence of growth factors, feeder layer optionally without animal serum and additives, and wherein said continuous diploid avian cell lines preferably do not produce replication-competent endogenous retrovirus particles;
j) optionally, further adapting said adherent avian EBx® cell lines to suspension culture conditions;
k) Optionally further subcloning said avian EBx® cells, for example by limit dilution.

In a preferred embodiment, the present invention relates to a process for obtaining continuous diploid avian cell lines, named EBx®, derived from avian embryonic stem cells (ES), wherein said avian cell lines do not produce replication-competent endogenous retrovirus particles, and said process comprising the steps of:
a) isolating bird embryo(s) at a developmental stage around oviposition, wherein the genome of said bird does not contain endogenous proviral sequences susceptible to produce replication competent endogenous retroviral particles;
b) suspending avian embryonic stem (ES) cells obtained by dissociating embryo(s) of step a) in a basal culture medium supplemented with at least:
   - Insulin Growth factor 1 (IGF-1) and Ciliary Neurotrophic factor (CNTF); and
   - mammalian serum such as foetal bovine serum;
c) seeding the suspension of ES cells obtained in step b) on a layer of feeder cells and further culturing the ES cells for at least one passage;
e) withdrawing IGF-1 and CNTF from the culture medium, and further culturing the cells for at least one passage;
f) progressively decreasing the concentration of feeder cells in the culture medium so as to obtain a total withdrawal of feeder layer after several passages, and further culturing the cells;
g) progressively decreasing the concentration of said mammalian serum in the culture medium so as to obtain a total withdrawal of mammalian serum after several passages and:
h) obtaining adherent avian EBx® cell lines derived from ES cells capable of proliferating in a basal medium in the absence of growth factors, feeder layer and mammalian serum, and wherein said continuous diploid avian cell lines do not produce replication-competent endogenous retrovirus particles;
i) optionally, further adapting adherent avian EBx® cell lines to suspension culture conditions, preferably by promoting the growth as suspension, more preferably by transferring the adherent avian EBx® cell lines obtained in step h) in another support having lower attachment characteristic than the initial support (i.e. such as Ultra Low attachment support).
Step j) of adapting adherent avian EBx® cell lines to suspension culture conditions, when carried out, can be effected in another preferred embodiment before the step g) of progressively decreasing the concentration of mammalian serum in the culture medium.

In another preferred embodiment, the basal culture medium in step b) of the process for obtaining continuous diploid avian cell lines according to the present invention, is further supplemented with a growth factor selected in the group comprising interleukin 6 (IL-6), interleukin 6 receptor (IL-6R), Stem cell Factor (SCF) and Fibroblast Growth Factor (FGF), and the said prcess further comprises a step d) of:
d) optionally withdrawing all the growth factors selected from the group comprising IL-6, IL-6R, SCF, FGF from the culture medium and further culturing the ES cells for at least one passage.

In a more preferred embodiment, when step d) is carried out, the step e)of withdrawing IGF-1 and CNTF from the culture medium, is effected after the step d) of withdrawing all the growth factors selected from the group comprising IL-6, IL-6R, SCF, FGF from the culture medium.

According to the invention, "basal culture medium" meant a culture medium with a classical media formulation that allows, by itself, at least cells survival, and even better, cell growth. Examples of basal media are BME (basal Eagle Medium), MEM (minimum Eagle Medium), medium 199, DMEM (Dulbecco's modified Eagle Medium), GMEM (Glasgow modified Eagle medium), DMEM-HamF12, Ham-F12 and Ham-F10, Iscove's Modified Dulbecco's medium, MacCoy's 5A medium, RPMI 1640, GTM3. Basal medium comprises inorganic salts (for examples: CaCl₂, KCl, NaCl, NaHCO₃, NaH₂PO₄, MgSO₄, ...), amino-acids, vitamins (thiamine, riboflavin, folic acid, D-Ca panthothenate, ...) and others components such as glucose, beta-mercapto-ethanol, sodium pyruvate. Preferably basal medium is a synthetic medium.

In addition, basal medium of the invention may be complemented with additives selected in the following group:
- 0.1 to 5 mM L-glutamine, preferably between 2 to 3 mM L-Glutamine;
- 0.05 to 2 mM sodium pyruvate, preferably between 0.1 mM to 1 mM sodium pyruvate;
- 0.1 to 2.5 % non-essential amino-acids, preferably around 1 % non-essential amino-acids;
- 0.05 to 5 mM beta-mercapto-ethanol, preferably around 0.16 mM beta-mercapto-ethanol;
- protein hydrolyzate of non-animal origin.

For the establishment of duck EBx® cells of the invention, the basal medium is preferably complemented with protein hydrolyzate of non-animal origin. Protein hydrolyzates of non-animal origin are selected from the group consisting bacteria tryptone, yeast tryptone, plant hydrolyzates, such as soy hydrolyzates, or a mixture thereof. In a preferred embodiment, the protein hydrolyzates of non-animal origin is yeast hydrolyzate. The term "hydrolyzate" includes an enzymatic digest of soy peptone or yeast extract. The hydrolysate can be obtained from a plurality of soy peptone or yeast extract preparations, respectively, which can be further enzymatically digested (for example, by papain), and/or formed by autolysis, thermolysis and/or plasmolysis. Hydrolysates also may be obtained commercially, such as Yeastolate, Hy-Soy, Hy-Yeast 412 and Hi-Yeast 444, from sources such as JRH BioSciences (Lenaxa, KA), Quest International (Norwich, N.Y.), OrganoTechnie S.A. (France) or Deutsche Hefewerke GmbH (Germany). Sources of yeast extracts also are disclosed in WO 98/15614. Sources of yeast extracts and soy hydrolysates also are disclosed in WO00/03000. The hydrolysates used in media of the invention are preferably purified from a crude fraction, because impurities which could interfere with efficient cultivation are preferably eliminated during this purification, thereby improving the consistency of the hydrolysate. Purification can be by ultrafiltration or Sephadex chromatography (for example, with Sephadex G25 or Sephadex G10 or equivalent materials), ion-exchange chromatography, affinity chromatography, size exclusion chromatography or "reversed-phase" chromatography. Preferably, purification is performed by ultrafiltration utilizing a 10kDa cut-off filter. These processes are known in the field. Using these methods, fractions can be selected which contain soy or yeast hydrolysate of defined molecular weight. Preferably, the average molecular weights of the soy and yeast hydrolysates are preferably between about 220 and 375 daltons. Preferably, yeast hydrolyzate is present in the cell culture medium. Yeast hydrolyzate 50X (around 200 g/l) obtained for example from JRH-BIOSCIENCES (Ref 58902) is present in the cell culture medium at a final concentration comprises between around 0.1X to 2X, preferably around 0.5X to around 1X into the culture medium. Soy hydrolyzate may also be added to the cell culture medium. Soy hydrolyzate 50X obtained for example from JRH-BIOSCIENCES (Ref 58903100M) is added at a final concentration comprises between around 0.1X to 2X, preferably around 1X into the culture medium. Alternatively a mixture of soy hydrolyzate and yeast hydrolyzate may be added to the cell culture medium as described in US 2004/0077086.

According to a preferred basal medium of the invention is DMEM-HamF12 that are complemented with 2 mM L-glutamin, 1 mM sodium pyruvate, 1 % non-essential amino-acids, 0.16 mM beta-mercapto-ethanol, and optionally with 1X yeast hydrolyzate.

By "complete culture medium", it is meant a basal culture medium complemented or not, preferably a basal synthetic medium, supplemented with at least one growth factor and animal serum. Example of complete culture medium is described in WO 03/076601, WO 05/007840, EP 787 180, US 6,114,168, US 5,340,740, US 6,656,479, US 5,830,510 and in Pain et al. (1996, Development 122:2339-2348). Alternatively, the complete culture medium may a conditioned medium, preferably BRL conditioned medium. By way of example, BRL conditioned media is prepared according to art-recognized techniques, such as described by Smith and Hooper (1987, Dev. Biol. 121:1-9). BRL cells are available from ATCC accession number CRL-1442. Conditioned medium may be supplemented with exogenous growth factors and animal serum as described below.

The term "growth factors" as used herein meant growth factor necessary for the survival and the growth of the undifferentiated avian ES cells in culture in a basal culture medium. It is possible to schematically distinguish two families of growth factors: the cytokines and the trophic factors. The cytokines are mainly cytokines whose action is through a receptor which is associated with the gp130 protein. Thus, leukemia inhibitory factor (LIF), interleukin 11, interleukin 6, interleukin 6 receptor, Ciliary Neurotrophic factor (CNTF), oncostatin and cardiotrophin have a similar mode of action with the recruitment at the level of the receptor of a specific chain and the combination of the latter with the gp130 protein in monomeric or sometimes hetero-dimeric form. The trophic factors are mainly Stem cell Factor (SCF), Insulin Growth factor 1 (IGF-1) and Fibroblast Growth Factor (FGF), preferably basic FGF (bFGF) or human FGF (hFGF).

The complete culture medium according to the invention comprises basal culture medium, preferably basal synthetic medium, and at least one cytokine whose action is through a receptor which is associated with the gp130 protein and/or at least one trophic factors. Preferably, the complete culture medium according to the invention comprises basal medium and at least one growth factor selected in the group consisting of Leukemia Inhibitory factor (LIF), oncostatin, cardiotrophin, Insulin Growth factor 1 (IGF-1), Ciliary Neurotrophic factor (CNTF), Interleukin 6 (IL-6), interleukin 6 receptor (IL-6R), Stem cell Factor (SCF), Fibroblast Growth Factor (FGF), interleukin 11 (IL-11). According to a first preferred embodiment, the complete culture medium is basal medium supplemented with animal serum and with at least IGF-1 and CNTF. According to a second preferred embodiment, the complete culture medium is basal medium supplemented with animal serum and at least IGF-1, CNTF, IL-6 and IL-6R. According to a third preferred embodiment, the complete culture medium is basal medium supplemented with animal serum and at least IGF-1, CNTF, IL-6, IL-6R, SCF, FGF. According to another embodiment, the complete culture medium is a conditioned culture medium comprising growth factors (i.e expressed by BRL or STO cells for example) and optionally supplemented with at least one exogenous growth factors selected in the group comprising: LIF, IGF-1,CNTF, IL-6, IL-6R, SCF, FGF, IL-11. The concentration of growth factors IGF-1, CNTF, IL-6, IL-6R, SCF, FGF, IL-11 in the basal medium or in the conditioned culture medium is comprised between about 0.01 to 10 ng/ml, preferably, 0.1 to 5 ng/ml, and more preferably about 1 ng/ml.

The culture medium of the invention may also comprise in addition antibiotics, such as for example, gentamycin, penicillin and streptomycin, to prevent bacterial contamination. Antibiotics may be added to the culture medium at the early passages of ES cells culture. For example, gentamycin at a final concentration of 10 ng/ml, penicillin at a final concentration of 100 U/ml and streptomycin at a final concentration of 100 µg/ml may be added to the culture medium. In a preferred embodiment, no antibiotics is added to the culture medium during the late steps of process of establishment of continuous diploid avian cell lines of the invention.

During the process of establishment of avian embryonic stem cells of the invention, the cells are cultured on a layer of feeder cells. More preferably, feeder cells are animal cells or cell lines cultured for the purpose of culturing avian ES cells. Alternatively, the feeder cells can be substituted with extra-cellular matrix plus bound growth factors. Feeder matrix will thereafter refers to either feeder cells or extra-cellular matrix. A feeder matrix as used herein is constructed in accordance with procedures known in the art. As noted above, it is preferred that the feeder matrix be preconditioned. By the term "preconditioned" it is meant that the feeder matrix is cultured in the presence of media for a period of time prior to the depositing of cells originating from the blastoderm disk fertilized avian eggs in contact with the feeder matrix, e.g. a time sufficient to initiate and establish production of, for example, growth factors or other factors by the feeder matrix; usually a feeder matrix is preconditioned by culturing the feeder matrix by itself for one to two days prior to the depositing of cells originating from the blastoderm disk fertilized avian eggs in contact with the feeder matrix. The feeder cells preferably comprises mouse fibroblast cells. STO fibroblasts are preferred, but primary fibroblasts are also suitable. Also while the present invention has been described with respect to the use of mouse cell feeder matrices, it is contemplated that feeder matrices comprising cells from other murine species (e.g. rat); other mammalian species (e.g; ungulate, bovine, porcine species); or avian species (e.g. Gallinacea, chicken, turkey, duck, goose, quail, pheasant) may also be used. In another embodiment, feeder cells of the invention may be transfected with expression vector(s) allowing for example the constitutive expression of growth factors such as avian SCF in STO cells. Thus, this "feeder" produces the factor in a form which is soluble and/or attached in the plasma membrane of the cells. Thus, the culturing process of the present invention may optionally comprise establishing a monolayer of feeder cells. Feeder cells are mitotically inactivated using standard techniques. For example, the feeder cells may be exposed to X or gamma radiation (e.g. 4000 Rads of gamma radiation) or may be treated with Mitomycin C (e.g. 10 µg/ml for 2-3 hours). Procedures for mitotically inactivating cells are also detailed in the information typically sent with cells from the American Type Culture Collection (ATCC), 10801 University Boulevard, Manassas, Va. 20110-2209 (e.g. STO feeder cells are available under ATCC accession number 1503). Mono-layers may optionally be cultured to about 80 % confluency, preferably to about 90 % confluency, and more preferably about 100 % confluency. While configuration of the feeder cells as a monolayer is the preferred configuration for the culture, any suitable configuration is contemplated to be within the scope of the present invention. Thus, for example, layers, mono-layers, clusters, aggregates or other associations or groupings of feeder cells are contemplated to fall within the scope of the present invention and are particularly contemplated to fall with the meaning of the term "matrix".

The culture medium of the invention is supplemented with animal serum. The animal serum preferably used is foetal animal serum. Foetal bovine serum is preferred. Also while the present invention has been described with respect to the use of foetal bovine serum, it is contemplated that animal serum comprising serum from other animal species (e.g. chicken, horse, porcine, ungulate, etc.) may also be used. The final concentration of animal serum in the culture medium is comprises between approximately 1 to 25 %, preferably between 5 % to 20 %, more preferably between 8% and 12 %. In the preferred embodiment, the final concentration of animal serum in the culture medium is approximately 10 %. According to a preferred embodiment, the culture medium comprises approximately 10 % of fetal calf serum.

The present invention also relates to the continuous diploid avian EBx® cell lines. EBx® cells are small, round (i;e diameter around 10 um), individualized cells with a doubling time of around 30 hours or less at 37°C or 39°C. The avian EBx® cells, preferably the duck EBx® or chicken EBx® ev-0, express an embryonic stem cell phenotype with the following characteristics:
- a high nucleo-cytoplasmic ratio,
- an endogenous telomerase activity,
- optionally, they may express one or more additional ES markers such as alkaline phosphatase, SSEA-1, EMA-1, ENS1 markers.
- A doubling time shorter than the doubling time of the avian ES cells of step a) of the process of the invention (48h to 72h at 39°C), of about 30 hours or less (preferably 24 hours) at 37°C.

Said cells EBx® preferably do not produce replication competent endogenous retrovirus particles. The avian EBx® cell lines of the invention are capable of proliferating indefinitely in a basal medium, in particular in a medium such as SAFC Excell media, DMEM, GMEM, DMEM-HamF12 or McCoy, free of exogenous growth factors, serum and/or inactivated feeder layer, optionally complemented with various additives commonly used by persons skilled in the art. Examples of additives are non-essential amino acids, vitamins, sodium pyruvate and antibiotics. Duck EBx® cells of the invention have the remarkable feature to grow in a basal culture medium that is not complemented with glutamine.

The karyotype analysis of avian EBx® cells of the invention shows that EBx® cells are diploid and genetically stable over the generations.

The present invention provides an avian EBx® cell, preferably a chicken EBx® or a duck EBx® cells, transfected with at least one expression vector comprising at least one expression cassette comprising nucleic acids sequence, preferably desoxy-ribonucleic acid (DNA) sequence, encoding a recombinant protein or polypeptide of interest operably linked to a promoter sequence capable of effecting expression of said protein in the cell. The expression vector further comprises at least one expression cassette comprising at least a DNA sequence encoding a selectable marker operably linked to a promoter sequence capable of effecting expression of said selectable marker in the host cell.

Expression vectors contain the necessary elements for the transcription and translation of at least one coding sequence of interest. Methods which are well known to and practiced by those skilled in the art can be used to construct expression vectors containing sequences encoding the proteins and polypeptides of interest, as well as the appropriate transcriptional and translational control elements. These methods include in vitro recombinant DNA techniques, synthetic techniques, and *in vivo* genetic recombination. Such techniques are described for example in Sambrook et al. (1989, Molecular Cloning, A Laboratory Manual, Cold Spring Harbor Press, Plainview, N.Y.) and in Ausubel et al. (1989, Current Protocols in Molecular Biology, John Wiley & Sons, New York, N.Y.).

Suitable expression vectors comprise at least one expression cassette that comprises nucleic acid sequence, preferably DNA sequence, encoding heterologous protein(s) of interest that are operably linked to control element and regulatory sequences. At least, the expression cassette comprises nucleic acid sequence, preferably DNA sequence, encoding heterologous protein(s) of interest that are operably linked to a promoter sequence. "Promoter" as used herein refers to a nucleic acid sequence that regulates expression of a gene. The term "operably linked" as used herein refers to the configuration of coding and control sequences, for example, within an expression vector, so as to achieve transcription and/or expression of the coding sequence. Thus, control sequences operably linked to a coding sequence are capable of effecting the expression of the coding sequence and regulating in which tissues, at what developmental time points, or in response to which signals, and the like, a gene is expressed. A coding sequence is operably linked to or under the control of transcriptional regulatory regions in a cell when DNA polymerase will bind the promoter sequence and transcribe the coding sequence into mRNA that can be translated into the encoded protein. The control sequences need not to be contiguous with the coding sequence, so long as they function to direct the expression thereof. Thus, for example, intervening untranslated or transcribed sequences can be present between a promoter sequence and the coding sequence and the promoter sequence can still be considered "operably linked" to the coding sequence. Such intervening sequences include but are not limited to enhancer sequences which are not transcribed or are not bound by polymerase. The term "expressed" or "expression" as used herein refers to the transcription of a nucleotide sequence into an RNA nucleic acid molecule at least complementary in part to a region of one of the two nucleic acid strands of a gene coding sequence and/or to the translation from an RNA nucleic acid molecule into a protein or polypeptide.

Such expression cassette further comprises control elements, or regulatory sequences which are those non-translated regions of the cassette, beside promoters (e. g. enhancers, 5' and 3' untranslated regions) that interact with host cellular proteins to carry out transcription and translation. As used herein, the term "control element" and "regulatory sequences" includes promoters, enhancers, and other elements that may control gene expression. Standard molecular biology textbooks such as Sambrook et al. eds "Molecular Cloning: A Laboratory Manual" 3rd ed., Cold Spring Harbor Press (2001) may be consulted to design suitable expression vectors that may further include an origin of replication and selectable gene markers. Such elements can vary in their strength and specificity. Depending on the vector system, any number of suitable transcription and translation elements, including constitutive and inducible promoters, can be used.

It should be recognized, however, that the choice of a suitable expression vector and the combination of functional elements therein depends upon multiple factors including for example the type of protein to be expressed. Representative examples of expression vectors include, for example, bacterial plasmid vectors including expression and cloning vectors such as, but not limited to, pBR322, animal viral vectors such as, but not limited to, modified avian adenovirus, measles virus, influenza virus, polio virus, pox virus, retrovirus, and the like and vectors derived from bacteriophage nucleic acid, for example, plasmids and cosmids, artificial chromosomes, such as but not limited to, Yeast Artificial Chromosomes (YACs) and Bacterial Artificial Chromosomes (BACs), and synthetic oligonucleotides like chemically synthesized DNA or RNA. Accordingly, the term "nucleic acid vector" or "vector" as used herein refers to a natural or synthetic single or double stranded plasmid or viral nucleic acid molecule, or any other nucleic acid molecule that can be transfected or transformed into cells and replicate independently of, or within, the host cell genome. A nucleic acid can be inserted into a vector by cutting the vector with restriction enzymes and ligating the pieces together. The nucleic acid molecule can be RNA or DNA. Preferably the nucleic acid molecule is DNA. Certain vectors are capable of autonomous replication in a host cell into which they are introduced, for example, bacterial vectors having a bacterial origin of replication and episomal mammalian vectors. Other vectors, such as non-episomal mammalian vectors, are integrated into the genome of a host cell upon introduction into the host cell, and thereby are replicated along with the host genome.

The expression cassette for use in protein expression system are designed to contain at least one DNA sequence encoding a recombinant protein of interest operably linked to a promoter sequence, and optionally control element or regulatory sequence. Control element or regulatory sequences are necessary or required for proper transcription and regulation of gene expression. These sequences are preferably selected in the group consisting of transcriptional initiation and termination sequences, enhancer, intron, origin of replication sites, polyadenylation sequences, peptide signal and chromatin insulator elements. Regulatory sequences are described, for example, in Goeddel; Gene Expression Technology: Methods in Enzymology 185, Academic Press, San Diego, Calif. (1990). Enhancer sequences may be located upstream or downstream of promoter region sequences for optimizing gene expression. An "enhancer" is a nucleotide sequence that acts to potentiate the transcription of genes independently of the identity of the gene, the position of the sequence in relation to the gene, or the orientation of the sequence. The vectors of the present invention optionally include enhancers. Example of enhancers are CMV immediate early enhancer and SV40 early enhancer.

An intronic sequence may be located upstream or downstream of sequence encoding a recombinant protein of interest for optimizing gene expression. According to a preferred embodiment, the intronic sequence is located between the promoter sequence and the sequence encoding recombinant protein of interest. The intronic sequence of the invention is preferably selected in the group consisting of a chimeric intron composed of 5'-donor site from the first intron of human beta-globin gene and the branch and 3'-acceptor site from the intron of an immunoglobulin gene heavy chain variable region.

The promoter sequences of the invention are preferably selected from genes of mammalian or avian origin or from mammalian or avian viral genes. In a preferred embodiment, the promoter sequence is from viral origin, and is selected in the group consisting of human or murine cytomegalovirus (CMV) promoter, avian sarcoma virus (ASV)xLN promoter, early and late promoters from simian virus 40 (SV40) (Fiers et al. (1973) Nature 273:113), Rous Sarcoma Virus (RSV) promoter, Herpes Simplex virus (HSV) thymidine kinase promoter, respiratory syncytial virus promoter, MDOT promoter, polyoma virus promoter, adenovirus 2 promoter, bovine papilloma virus promoter, adenovirus major late promoter and functional portions of each of these promoters. According to another embodiment, the promoter sequence is selected in the group consisting of murine phospho-glycerate kinase promoter, murine leukaemia virus (MLV), mouse mammary tumor virus (MMTV), EiF4alpha promoter, chimeric EF1alpha/HTLV promoter, chimeric CAG promoter (composite promoter that combines human CMV immediate early enhancer and a modified chicken beta-actin promoter and first intron) and avian gene promoters and functional portions of each of these promoters. Among avian gene promoters, the promoter is preferably selected among chicken promoters such as beta-actin promoter, oviduct-specific promoter, ovomucoid promoter, ovalbumin promoter, conalbumin promoter, ovomucin promoter, ovotransferrin promoter, lysozyme promoter, ENS1 gene promoter and functional portions of each of these promoters.

According to another embodiment, promoters may be selected among regulated promoters such promoters that confer inducibility by particular compounds or molecules, e. g., the glucocorticoid response element (GRE) of mouse mammary tumor virus (MMTV) is induced by glucocorticoids (Chandler et al. (1983) Cell 33: 489-499). Also, tissue-specific promoters or regulatory elements can be used (Swift et al. (1984) Cell, 38: 639-646), if necessary or desired. Non-limiting examples of other promoters which may be useful in the present invention include, without limitation, Pol III promoters (for example, type 1, type 2 and type 3 Pol III promoters) such as HI promoters, U6 promoters, tRNA promoters, RNase MPR promoters and functional portions of each of these promoters. Typically, functional terminator sequences are selected for use in the present invention in accordance with the promoter that is employed.

The expression vector of the invention may further comprises at least one expression cassette comprising at least a nucleic acid sequence, preferably a DNA sequence, encoding a selectable marker operably linked to a promoter sequence capable of effecting expression of said selectable marker in the cell. Such selectable marker may confer resistance to the EBx® cell harboring the vector to allow their selection in appropriate selection medium. For the methods of this invention, stable expression is generally preferred to transient expression because it typically achieves more reproducible results and also is more amenable to large scale production. Accordingly, the expression vectors of the invention are stably incorporated into the chromosomal DNA of the EBx® cell. Following the introduction of foreign DNA, engineered cells may be allowed to grow for 1-2 days in an enriched media, and then are switched to a selective media. The selectable marker in the recombinant vector confers resistance to the selection and allows selection of cells which have stably integrated the vector into their chromosomes and grow to form foci which in turn can be cloned and expanded into cell lines.

In a preferred embodiment, anti-metabolite resistance is used as the basis of selection for the following non-limiting examples of marker genes: DHFR, which confers resistance to methotrexate (Wigler et al. (1980) Proc. Natl. Acad. Sci. USA, 77:357; and O'Hare et al. (1981) Proc. Natl. Acad. Sci. USA, 78:1527); GPT, which confers resistance to mycophenolic acid (Mulligan and Berg (1981) Proc. Natl. Acad. Sci. USA, 78:2072); NEO, which confers resistance to the amino-glycoside G418 (Wu and Wu (1991) Biotherapy, 3:87-95; Tolstoshev (1993) Ann. Rev. Pharmacol.Toxicol., 32:573-596; Mulligan (1993) Science, 260: 926- 932; Anderson (1993) Ann. Rev. Biochem., 62:191-21); and Hygromycin B, which confers resistance to hygromycin (Santerre et al. (1984) Gene, 30:147). In a most preferred embodiment, antibiotic resistance genes are used as the basis of selection. According to a preferred embodiment the selectable marker of the invention is neomycin resistance gene. Preferably, the nucleic acid sequence encoding NEO is the neomycin/kanamycin resistance gene of TN5. According to another preferred embodiment the selectable marker of the invention is kanamycin resistance gene. According to another preferred embodiment the selectable marker of the invention is puromycin resistance gene.

Alternatively, such selection systems require that EBx® cells of the invention are previously genetically modified to display the appropriate genotype (i.e TK-, HGPRT-, ART-, DHFR-, GPT-, etc.). A number of selection systems can be used, including but not limited to, the Herpes Simplex Virus thymidine kinase (HSV TK), (Wigler et al. (1977) Cell 11:223), hypoxanthine-guanine phosphoribosyl transferase(HGPRT) (Szybalska & Szybalski (1992) Proc. Natl. Acad. Sci. USA, 48:202), and adenine phosphor-ribosyl transferase (Lowy et al. 1980 Cell 22: 817) genes, which can be employed in tk-, hgprt-, or art-cells (APRT) respectively. Methods commonly known in the art of recombinant DNA technology can be routinely applied to select the desired recombinant cell clones, and such methods are described, for example, in Ausubel *et al.* (1993) and Kriegle (1990, Gene Transfer and Expression, A Laboratory Manual, Stockton Press, NY; in Chapters 12 and 13).

In addition, the expression levels of the expressed protein molecule can be increased by vector amplification (for a review, see Bebbington & Hentschel, "The use of vectors based on gene amplification for the expression of cloned genes in mammalian cells in DNA cloning",Vol.3, Academic Press, New-York 1987). When a marker in the vector system expressing a protein is amplifiable, an increase in the level of inhibitor present in the host cell culture will increase the number of copies of the marker gene. Since the amplified region is associated with the protein-encoding gene, production of the protein will concomitantly increase (Crouse et al. (1983), Mol. Cell. Biol., 3: 257). Vectors which harbor glutamin synthase (GS) or dihydrofolate reductase (DHFR) encoding nucleic acid as the selectable markers can be amplified in the presence of the drugs methionine sulphoximine or methotrexate, respectively. A glutamin synthase expression system and components thereof are detailed in PCT publications: W087/04462; WO 86/05807; WO 89/01036; WO 89/10404; and WO 91/06657. In addition, glutamine synthase expression vectors that can be used in accordance with the present invention are commercially available from suppliers, including, for example, Lonza Biologics, Inc. (Portsmouth, NH).

The host cells which contain the coding sequence and which express the biologically active gene products (i.e protein of interest, selectable marker, ...) may be identified by at least four general approaches; (a) DNA-DNA or DNA-RNA hybridization; (b) the presence or absence of "marker" gene functions; (c) assessing the level of transcription as measured by the expression of the respective mRNA transcripts in the EBx® cell; and (d) detection of the gene product as measured by immuno-assay or by its biological activity. In the first approach, the presence of the coding sequence of the protein of interest inserted in the expression vector can be detected by DNA-DNA or DNA-RNA hybridization using probes comprising nucleotide sequences that are homologous to the respective coding sequences, respectively, or portions or derivatives thereof. In the second approach, the recombinant expression vector/host system can be identified and selected based upon the presence or absence of certain "marker" gene functions (e. g., thymidine kinase activity, resistance to antibiotics, resistance to methotrexate, etc.).

One or both of the vectors of the invention may comprises at least one origin of replication to allow for the replication of vector constructs inside the host cells. According to a preferred embodiment, vectors of the invention comprise one bacterial origin of replication, such as F1 ORI to allow for the replication of expression vector in bacteria (for example E.coli) and a SV40 origin of replication to allow for the expression vector to be checked by rapid transient assay.

The expression vector of the invention may further comprises chromatin insulator elements. Chromatin insulator elements of the invention include boundary elements (BEs), matrix attachment regions (MARs), locus control regions (LCRs), and universal chromatin opening elements (UCOEs). Boundary elements ("BEs"), or insulator elements, define boundaries in chromatin in many cases (Bell & Felsenfeld (1999) Curr Opin Genet Dev 9:191-198) and may play a role in defining a transcriptional domain *in vivo*. BEs lack intrinsic promoter/enhancer activity, but rather are thought to protect genes from the transcriptional influence of regulatory elements in the surrounding chromatin. The enhancer-block assay is commonly used to identify insulator elements. In this assay, the chromatin element is placed between an enhancer and a promoter, and enhancer-activated transcription is measured. Boundary elements have been shown to be able to protect stably transfected reporter genes against position effects in Drosophila, yeast and in mammalian cells (Walters et al. (1999) Mol Cell Biol 19:3714-3726). Matrix Attachment Regions ("MARs"; also known as Scaffold Attachment Regions or Scaffold/Matrix Attachment Regions ("S/MARs")) are DNA sequences that bind isolated nuclear scaffolds or nuclear matrices in vitro with high affinity (Hart and Laemmli (1998) Curr Opin. Genet Dev 8:519-525). As such, they may define boundaries of independent chromatin domains, such that only the encompassing cis-regulatory elements control the expression of the genes within the domain. MAR elements can enhance expression of heterologous genes in cell culture lines (Kalos and Fournier (1995) Mol Cell Biol 15:198-207)). Locus control regions ("LCRs") are cis-regulatory elements required for the initial chromatin activation of a locus and subsequent gene transcription in their native locations (reviewed in Grosveld 1999, Curr Opin Genet Dev 9:152-157). The most extensively characterized LCR is that of the globin locus. Ubiquitous chromatin opening elements ("UCOEs", also known as "ubiquitously acting chromatin opening elements") have recently been reported (See WO00/05393). According to a preferred embodiment, the chromatin insulator element of the invention is a MAR element. Preferably, the MAR element is selected among chicken lysozyme 5'MAR elements as described in WO 02/074969 or human MAR elements as described in WO 2005/040377.

As will be appreciated by those skilled in the art, the selection of the appropriate vector, e. g., plasmid, components for proper transcription, expression (promoter, control sequences & regulatory sequence), and isolation of proteins produced in cell expression systems is known and routinely determined and practiced by those having skill in the art.

According to one embodiment, the EBx® cells of the invention are transfected with at least one expression vector wherein said expression vector comprises at least:
- a first expression cassette comprising the following DNA sequences in the following order: CMV promoter sequence of SEQ ID N° 1 or a fragment or a variant thereof, intronic sequence, DNA sequence encoding a recombinant protein of interest, polyadenylation sequence; and
- a second expression cassette comprising the following DNA sequences in the following order: SV40 promoter, antibiotic resistance gene (preferably Neomycin resistance gene), poly-adenylation sequence;
- optionally, at least one chicken lysozyme 5'MAR element as described in WO 02/074969 or a human MAR elements as described in WO 2005/040377.

According to second embodiment, the EBx® cells of the invention are transfected with at least one expression vector wherein said expression vector comprises at least:
- a first expression cassette comprising the following DNA sequences in the following order: chimeric EF1alpha/HTLV promoter sequence of SEQ ID N° 2 or a fragment or a variant thereof, intronic sequence, DNA sequence encoding a recombinant protein of interest, polyadenylation sequence; and
- a second expression cassette comprising the following DNA sequences in the following order: SV40 promoter, antibiotic resistance gene (preferably Neomycin resistance gene), polyadenylation sequence;
- optionally, at least one chicken lysozyme 5'MAR element as described in WO 02/074969 or a human MAR elements as described in WO 2005/040377.

According to one embodiment, the EBx® cells of the invention are transfected with at least one expression vector wherein said expression vector comprises at least:
- a first expression cassette comprising the following DNA sequences in the following order: RSV promoter sequence of SEQ ID N° 3 or a fragment or a variant thereof, intronic sequence, DNA sequence encoding a recombinant protein of interest, polyadenylation sequence; and
- a second expression cassette comprising the following DNA sequences in the following order: SV40 promoter, antibiotic resistance gene (preferably Neomycin resistance gene), poly-adenylation sequence;
- optionally, at least one chicken lysozyme 5'MAR element as described in WO 02/074969 or a human MAR elements as described in WO 2005/040377.

According to a preferred embodiment, the intronic sequence is the sequence of SEQ ID N° 4 or a fragment or a variant thereof and the poly-adenylation sequence is the sequence of SEQ-ID N° 5 or a fragment or a variant thereof.

When the recombinant protein of interest of the invention is a multimeric protein, the different chains of said protein are either encoded in a single expression vector, or in different expression vectors. In the latter case, the different expression vectors are co-transfected either simultaneously or successively into the EBx® cell. "Co-transfection" means the process of transfecting EBx® cell with more than one expression vectors. When the cell has been co-transfected with an expression vector capable of expressing the light chain of the antibody and a vector capable of expressing the heavy chain of the antibody, the vector preferably contain independently selectable markers. When a single expression vector is capable of expressing the light chain of the antibody and the heavy chain of the antibody, the vector preferably contain at least one selectable marker.

Surprisingly, the inventors have now found that the level of antibody expression in EBx® cells is higher when transfecting a single expression vector encoding the heavy chain and light chains of an antibody, compared to the co-transfection of two expression vectors, each of them encoding one antibody chain. Furthermore, the inventors also found that, when transfecting a single vector, the level of antibody expression in EBx® cells is higher when the expression vector is comprising in the following order: a first cassette encoding the heavy chain of an antibody and the second cassette encoding the light chain of the antibody, each cassette having the same promoter. Indeed the balanced expression of the light and heavy chains of an antibody from EBx® cells is desirable given that the light and heavy chains are linked together in the antibody molecule in equimolar proportions. The expression vector allows the antibody to be obtained in functional form and to be secreted in good yields. Thus the process enables sufficient quantities of functional antibody to be obtained for use in the immunotherapy of pathological disorders.

The cell line of the present invention is capable of producing all kinds of antibodies that generally comprise equimolar proportions of light and heavy chains. The invention therefore includes human antibodies wherein the amino acid sequences of the heavy and light chains are homologous with those sequences of antibodies produced by human lymphocytes in vivo or in vitro by hybridomas. Also included in the invention are altered antibodies such as hybrid antibodies in which the heavy and light chains are homologous to a natural antibody but are combined in a way that would not occur naturally. For example, a bispecific antibody has antigen binding sites specific to more than one antigen. The constant region of the antibody may relate to one or other of the antigen binding regions or may be from a further antibody. Altered antibodies, such as chimeric antibodies have variable regions from one antibody and constant regions from another. Thus, chimeric antibodies may be species/species chimaeras or class/class chimaeras. Such chimeric antibodies may have one or more further modifications to improve antigen binding ability or to alter effector functioning. Another form of altered antibody is a humanized or CDR-grafted antibody including a composite antibody, wherein parts of the hypervariable regions in addition to the CDRs are transferred to the human framework. Additional amino acids in the framework or constant regions of such antibodies may be altered. Included in the definition of altered antibody are Fab fragments which are roughly equivalent to the Y branch portions of the heavy and light chains; these may include incomplete fragments or fragments including part of the Fc region. Thus, within the scope of the invention is included, any altered antibody in which the amino acid sequence is not one which exists in nature.

Preferably, the protein of interest is a monoclonal antibody, preferably a human monoclonal antibody, or an altered antibody, and the EBx® cell of the invention are transfected with at least one expression vector wherein said expression vector comprises at least in the following order :
- a first expression cassette comprising the following DNA sequences in the following order: promoter sequence, intronic sequence, DNA sequence (preferably cDNA sequence) encoding the heavy chain of an antibody or a fragment thereof, polyadenylation sequence;
- a second expression cassette comprising the following DNA sequences in the following order: promoter sequence, intronic sequence, DNA sequence (preferably cDNA sequence) encoding the light chain of the antibody or a fragment thereof, polyadenylation sequence;
- a third expression cassette comprising the following DNA sequences in the following order: viral promoter, antibiotic resistance gene, polyadenylation sequence;
- optionally, at least one chicken lysozyme 5'MAR element as described in WO 02/074969 or a human MAR elements as described in WO 2005/040377.

However, it is also an object of the instant invention to provide an EBx® cell transfected with at least one expression vector wherein said expression vector comprises at least in the following order:
- a first expression cassette comprising the following DNA sequences in the following order: promoter sequence, intronic sequence, DNA sequence (preferably cDNA sequence) encoding the light chain of an antibody or a fragment thereof, polyadenylation sequence;
- a second expression cassette comprising the following DNA sequences in the following order: promoter sequence, intronic sequence, DNA sequence (preferably cDNA sequence) encoding the heavy chain of the antibody or a fragment thereof, polyadenylation sequence;
- a third expression cassette comprising the following DNA sequences in the following order: viral promoter, antibiotic resistance gene, polyadenylation sequence;
- optionally, at least one chicken lysozyme 5'MAR element as described in WO 02/074969 or a human MAR elements as described in WO 2005/040377.

According to another preferred embodiment, the EBx® cell of the invention is transfected with at least one expression vector wherein said expression vector comprises at least in the following order:
- a first expression cassette comprising the following DNA sequences in the following order: CMV promoter of SEQ ID N°1 or a fragment or a variant thereof, intronic sequence, cDNA sequence encoding the heavy chain of an antibody or a fragment thereof, polyadenylation sequence;
- a second expression cassette comprising the following DNA sequences in the following order: CMV promoter of SEQ ID N° 1 or a fragment or a variant thereof, intronic sequence, cDNA sequence encoding the light chain of the antibody or a fragment thereof, polyadenylation sequence;
- a third expression cassette comprising the following DNA sequences in the following order: SV40 promoter, neomycin resistance gene, polyadenylation sequence;
- optionally, at least one chicken lysozyme 5'MAR element as described in WO 02/074969 or a human MAR elements as described in WO 2005/040377.

According to another preferred embodiment, the EBx® cell of the invention is transfected with at least one expression vector wherein said expression vector comprises at least in the following order:
- a first expression cassette comprising the following DNA sequences in the following order: chimeric EF1alpha/HTLV promoter of SEQ ID N° 2 or a fragment or a variant thereof, intronic sequence, cDNA sequence encoding the heavy chain of an antibody or a fragment thereof, polyadenylation sequence;
- a second expression cassette comprising the following DNA sequences in the following order: chimeric EF1alpha/HTLV promoter of SEQ ID N°2 or a fragment or a variant thereof, intronic sequence, cDNA sequence encoding the light chain of the antibody or a fragment thereof, polyadenylation sequence;
- a third expression cassette comprising the following DNA sequences in the following order: SV40 promoter, neomycin resistance gene, polyadenylation sequence;
- optionally, at least one chicken lysozyme 5'MAR element as described in WO 02/074969 or a human MAR elements as described in WO 2005/040377.

According to another preferred embodiment, the EBx® cell of the invention is transfected with at least one expression vector wherein said expression vector comprises at least in the following order:
- a first expression cassette comprising the following DNA sequences in the following order: RSV promoter of SEQ ID N° 3 or a fragment or a variant thereof, intronic sequence, cDNA sequence encoding the heavy chain of an antibody or a fragment thereof, polyadenylation sequence;
- a second expression cassette comprising the following DNA sequences in the following order: RSV promoter of SEQ ID N° 3 or a fragment or a variant thereof, intronic sequence, cDNA sequence encoding the light chain of the antibody or a fragment thereof, polyadenylation sequence;
- a third expression cassette comprising the following DNA sequences in the following order: SV40 promoter, neomycin resistance gene, polyadenylation sequence;
- optionally, at least one chicken lysozyme 5'MAR element as described in WO 02/074969 or a human MAR elements as described in WO 2005/040377.

The light and heavy chain genes may constitute genomic DNA or, preferably, cDNA, and are cloned using procedures known in the art (Molecular Cloning: A Laboratory Manual, Second Edition, Maniatis et al, Cold Spring Harbor).

According to another embodiment, the EBx® cell of the invention further comprises an expression vector comprising at least an expression cassette comprising nucleic acid sequence, preferably DNA sequence, encoding an anti-apoptotic protein operably linked to a promoter sequence capable of effecting expression of said anti-apoptotic protein in the cell. The anti-apoptotic protein is selected in the group comprising mammalian, avian, amphibian and fish Bcl2 family proteins. According to a preferred embodiment, the anti-apoptotic protein is an avian Bc12 family member named NR13 (Lee et al. 1999 Genes & Dev. 13:718-728; Lalle et al. 2002, Biochem. J. 368: 213-221). Example of expression vector encoding anti-apoptotic NR13 protein comprises at least:
- a first expression cassette comprising the following DNA sequences in the following order: CMV, RSV or chimeric EF1alpha/HTLV promoter sequence, intronic sequence, cDNA sequence encoding anti-apoptotic NR13 protein, polyadenylation sequence; and
- a second expression cassette comprising the following DNA sequences in the following order: SV40 promoter, puromycine resistance gene, poly-adenylation sequence;
- optionally a third expression cassette comprising the following DNA sequences in the following order: SV40 promoter, neomycin resistance gene, poly-adenylation sequence;
- optionally, a chicken lysozyme 5'MAR element as described in WO 02/074969 or a human MAR elements as described in WO 2005/040377.

According to a preferred embodiment the NR13 sequence is SEQ ID N° 6 or a fragment or a variant thereof.

The instant invention further provides a method for producing at least one biological product of interest in avian EBx® cell, said method comprising the steps of:
a) preparing EBx® cell according to the invention by transfection with at least one expression vector; transfection may be performed either on adherent or suspension EBx® cells;
b) culturing said transfected EBx® cell under suitable conditions and in a cell culture medium; and
c) harvesting the biological product of interest from said transfected EBx® cell, the cell culture medium, or both said EBx® cell and said medium.

The expression vectors described herein can be introduced into EBx® cells by a variety of methods. In particular, standard transfection procedures, well-known from the man skilled in the art may be carried out, such as calcium phosphate precipitation, DEAE-Dextran mediated transfection, electroporation, nucleofection (AMAXA Gmbh, GE), liposome-mediated transfection (using lipofectin® or lipofectamine® technology for example) or microinjection. According to a preferred embodiment, in the step a), EBx® cells, preferably chicken or duck EBx® cells are transfected by electropration, more preferably by nucleofection which is an optimized electropartaion technology developed by AMAXA Gmbh (DE), with at least one expression vector in adherent culture in a serum-free cell culture medium. According to a preferred embodiment, in the step a), EBx® cells, preferably chicken or duck EBx® cells are transfected by electropration, more preferably by nucleofection, with at least one expression vector in suspension culture in a serum-free cell culture medium. According to another preferred embodiment, in the step a), EBx® cells, preferably chicken or duck EBx® cells are transfected by liposome-mediated transfection, using compound like Lipofectamin® and the like, with at least one expression vector in adherent or in suspension culture in a serum-free cell culture medium.

As used herein the terms "protein of interest" refers to a polymer of amino acids, linked through peptide bonds. The term "protein of interest" includes proteins, protein fragments, protein analogues, polypeptides, oligopeptides, peptides and the like. According to the invention, by the term "biological product of interest" it is meant either a monomeric "protein of interest" or a composition of at least two monomeric proteins of interest to constitute a multimeric protein. Examples of multimeric protein of interest are antibodies that are composed of 4 monomeric proteins of interest (i.e two heavy and two light chains). "Protein" or "biological product" that is not naturally part of the EBx® cell genome are referred to as "heterologous protein" or "heterologous biological product".

The invention provides a biological product of interest having avian EBx® glycosylation. More specifically, the invention provides an antibody having chicken EBx® glycosylation, more preferably EB14 or EBv13 glycosylation, or having duck EBx® glycosylation, more preferabaly EB24 glycosylation, EB24-12 glycosylation, EB26 glycosylation, EB66 glycosylation.

The culturing of said transfected EBx® cells can be performed according to the cell culture techniques well-known by the man skilled in the art. For the purposes of understanding, yet without limitation, it will be appreciated by the skilled practitioner that cell cultures and culturing runs for protein production can include three general types; namely, continuous culture, batch culture and fed-batch culture. In a continuous culture, for example, fresh culture medium supplement (i.e. feeding medium) is provided to the cells during the culturing period, while old culture medium is removed daily and the product is harvested, for example, daily or continuously. In continuous culture, feeding medium can be added daily and can be added continuously, i.e., as a drip or infusion. For continuous culturing, the cells can remain in culture as long as is desired, so long as the cells remain alive and the environmental and culturing conditions are maintained. In batch culture, cells are initially cultured in medium and this medium is neither removed, replaced, nor supplemented, i.e., cells are not "fed" with new medium, during or before the end of the culturing run. The desired product is harvested at the end of the culturing run. For fed-batch cultures, the culturing run time is increased by supplementing the culture medium one or more times daily (or continuously) with fresh medium during the run, i.e., the cells are "fed' with new medium ("feeding medium") during the culturing period. Fed-batch cultures can include the various feeding regimens and times as described above, for example, daily, every other day, every two days, etc., more than once per day, or less than once per day, and so on. Further, fed-batch cultures can be fed continuously with feeding medium. The desired product is then harvested at the end of the culturing/production run. The present invention preferably embraces fed-batch cell cultures. According to the present invention, cell culture can be carried out, and proteins, preferably glycoproteins, can be produced by cells, under conditions for the large or small scale production of proteins, using culture vessels and/or culture apparatuses that are conventionally employed for animal or mammalian cell culture. As is appreciated by those having skill in the art, tissue culture dishes, T-flasks and spinner flasks are typically used on a laboratory scale. For culturing on a larger scale (e. g. 3L, 7L, 20L, 100L, 500 L, 5000 L, and the like), procedures including, but not limited to, a fluidized bed bioreactor, a hollow fiber bioreactor, roller bottle culture, or stirred tank bioreactor systems can be used. Microcarriers may or may not be used with the roller bottle or stirred tank bioreactor systems. The systems can be operated in a batch, continuous, or fed-batch mode. In addition, the culture apparatus or system may or may not be equipped with a cell separator using filters, gravity, centrifugal force, and the like.

In the cell culture processes or methods of this invention, the cells can be maintained in a variety of cell culture media. i.e. basal culture media, as conventionally known in the art. For example, the methods are applicable for use with large volumes of cells maintained in cell culture medium, which can be supplemented with nutrients and the like. Typically, "cell culturing medium" (also called "culture medium") is a term that is understood by the practitioner in the art and is known to refer to a nutrient solution in which cells, preferably animal or mammalian cells, are grown and which generally provides at least one or more components from the following: an energy source (usually in the form of a carbohydrate such as glucose); all essential amino acids, and generally the twenty basic amino acids, plus cysteine; vitamins and/or other organic compounds typically required at low concentrations; lipids or free fatty acids, e.g., linoleic acid; and trace elements, e.g., inorganic compounds or naturally occurring elements that are typically required at very low concentrations, usually in the micromolar range.

Cell culture medium can also be supplemented to contain a variety of optional components, such as hormones and other growth factors, e.g., insulin, transferrin, epidermal growth factor, serum, and the like ; salts, e.g., calcium, magnesium and phosphate, and buffers, e.g. HEPES; nucleosides and bases, e.g. adenosine, thymidine, hypoxanthine; and protein and tissue hydrolyzates, e.g., hydrolyzed animal protein (peptone or peptone mixtures, which can be obtained from animal byproducts, purified gelatin or plant material); antibiotics, e.g. gentamycin; and cell protective agents, e.g. Pluronic polyol (Pluronic F68). Preferred is a cell nutrition medium that is serum-free and free of products or ingredients of animal origin. EBx® cells of the invention have been adapted to the culture in serum-free conditions.

Commercially available media can be utilized and include, for example, Ham's F12 Medium (Sigma, St. Louis, MO), Dulbecco's Modified Eagles Medium (DMEM, Sigma), optipro medium (Invitrogen, Carlsbad, CA), or Excell media (SAFC, Lenexa, KS). To the foregoing exemplary media can be added the above-described supplementary components or ingredients, including optional components, in appropriate concentrations or amounts, as necessary or desired, and as would be known and practiced by those having in the art using routine skill. In addition, cell culture conditions suitable for the methods of the present invention are those that are typically employed and known for batch, fed-batch, or continuous culturing of cells, with attention paid to pH, e.g. about 6.5 to about 7.5 ; dissolved oxygen (O₂), e.g., between about 5-90% of air saturation and carbon dioxide (CO₂), agitation and humidity, in addition to temperature.

Once harvest the biological product of interest is usually concentrated. Once obtained in concentrated form, any standard technique, such as preparative disc gel electrophoresis, ion-exchange chromatography, gel filtration, size separation chromatography, isoelectric focusing and the like may be used to purify, isolate, and/or to identify the heterologous protein. Those skilled in the art may also readily devise affinity chromatographic means of heterologous protein purification, especially for those instances in which a binding partner of the heterologous protein is known, for example, antibodies. Isolation of monoclonal antibodies is simplified and safety is enhanced due to the absence of additional human or animal proteins in the culture. The absence of serum further increases reliability of the system since use of synthetic media, as contemplated herein, enhances reproducibility.

Examples of proteins of interest that can be advantageously produced by the method of this invention include, without limitation, cytokines, cytokine receptors, growth factors (e.g. EGF, HER-2, FGF-alpha, FGF-beta, TGF-alpha, TGF-beta, PDGF, IGF-1, IGF-2, NGF), growth factor receptors, including fragment of the protein thereof. Other non-limiting examples include growth hormones (e.g. human growth hormone, bovine growth hormone); insulin (e.g., insulin A chain and insulin B chain), pro-insulin, erythropoietin (EPO), colony stimulating factors (e.g. G-CSF, GM-CSF, M-CSF); interleukins (e.g. IL-1 through IL-12); vascular endothelial growth factor (VEGF) and its receptor (VEGF-R), interferons (e.g. IFN-alpha, beta and gamma), tumor necrosis factor (TNF) and their receptors (TNFR-1 and TNFR-2), thrombopoietin (TPO), thrombin, brain natriuretic peptide (BNP); clotting factors (e.g. FactorVIII, Factor IX, von Willebrands factor and the like), anti-clotting factors; tissue plasminogen activator (TPA), urokinase, follicle stimulating hormone (FSH), luteinizing hormone (LH), calcitonin, CD proteins (e. g., CD2, CD3, CD4, CD5, CD7, CD8, CD11a, CD11b, CD18, CD19, CD25, CD33, CD44, CD45, CD71, etc.), CTLA proteins (e.g.CTLA4); T-cell and B-cell receptor proteins, bone morphogenic proteins (BNPs, e.g. BMP-1, BMP-2, BMP-3, etc.), neurotrophic factors, e.g. bone derived neurotrophic factor (BDNF), neurotrophins, e.g. rennin, rheumatoid factor, RANTES, albumin, relaxin, macrophage inhibitory protein (e.g. MIP-1, MIP-2), viral proteins or antigens, surface membrane proteins, ion channel proteins, enzymes, regulatory proteins, antibodies, immunomodulatory proteins, (e.g. HLA, MHC, the B7 family), homing receptors, transport proteins, superoxide dismutase (SOD), G-protein coupled receptor proteins (GPCRs), neuromodulatory proteins, Alzheimer's Disease associated proteins and peptides, (e.g. A-beta) and others as known in the art. Fusion proteins and polypeptides, chimeric proteins and polypeptides, as well as fragments or portions, or mutants, variants, or analogs of any of the aforementioned proteins and polypeptides are also included among the suitable proteins, polypeptides and peptides that can be produced by the methods of the present invention.

In a preferred embodiment, the protein of interest is a glycoprotein, and preferably a viral protein. Example of viral proteins (subunits) that can be produced in the methods according to the invention include, without limitation, proteins from enterovirus, such as rhinovirus, aphtovirus, or poliomyelitis virus, herpes virus, such as herpes simplex virus, pseudorabies virus or bovine herpes virus, orthomyxovirus such as influenza virus, a paramyxovirus, such as newcastle disease virus, respiratory syncitio virus, mumps virus or a measles virus, retrovirus, such as human immunodeficiency virus or a parvovirus or a papovavirus, rotavirus or a coronavirus, such as transmissable gastroenteritisvirus or a flavivirus, such as tick-borne encephalitis virus or yellow fever virus, a togavirus, such as rubella virus or eastern-, western-, or venezuelean equine encephalomyelitis virus, a hepatitis causing virus, such as hepatitis A or hepatitis B virus, a pestivirus, such as hog cholera virus or a rhabdovirus, such as rabies virus. According to another embodiment, the protein of interest is a bacterial protein.

In another preferred embodiment, the biological product of interest is an antibody. The term "antibody" as used herein refers to polyclonal and monoclonal antibodies and fragments thereof, and immunologic binding equivalents thereof. The term "antibody" refers to a homogeneous molecular entity, or a mixture such as a polyclonal serum product made up of a plurality of different molecular entities, and broadly encompasses naturally-occurring forms of antibodies (for example, IgD, IgG, IgA, IgM, IgE) and recombinant antibodies such as single-chain antibodies, chimeric and humanized antibodies and multi-specific antibodies. The term "antibody" also refers to fragments and derivatives of all of the foregoing, and may further comprises any modified or derivatised variants thereof that retains the ability to specifically bind an epitope.

Antibody derivatives may comprise a protein or chemical moiety conjugated to an antibody. A monoclonal antibody is capable of selectively binding to a target antigen or epitope. Antibodies may include, but are not limited to polyclonal antibodies, monoclonal antibodies (mAbs), humanized or chimeric antibodies, camelized antibodies, single chain antibodies (scFvs), Fab fragments, F(ab')₂ fragments, disulfide-linked Fvs (sdFv) fragments, anti-idiotypic (anti-Id) antibodies, intra-bodies, synthetic antibodies, and epitope-binding fragments of any of the above. The term "antibody" also refers to fusion protein that includes a region equivalent to the Fc region of an immunoglobulin.

The present invention provides a method for the production of an antibody which comprises culturing a transfected EBx® cell of the present invention. Culture of the EBx® cells may be carried out in serum-containing or preferably serum and protein free media. The resulting antibody may be purified and formulated in accordance with standard procedures. Expression of both chains of Mabs in substantially equimolar proportions enables optimum yields of functional antibody to be obtained. The Transfected EBx® cells of the invention, and more specifically chicken EBx® and duck EBx® cells, are able to produce at least 10 pg/cell/day of immunoglobulin in batch culture, preferably at least 15 pg/cell/day of immunoglobulin in batch culture, more preferably at least 25 pg/cell/day of immunoglobulin in batch culture, even more preferably at least 35 pg/cell/day of immunoglobulin in batch culture. The two chains assemble within the cell and are then secreted into the culture medium as functional antibody. Antibody glycosylated by EBx® cells maintain antigen binding capability and effector functionality. Interestingly, the inventors have now demonstrated that the antibody, the antibody fragment, or the fusion proteins that include a region equivalent to a Fc region of an immunoglobulin, produced by the method of the invention have increased Fc-mediated cellular toxicity. For example, antibody of IgG1 subtype, produced in avian EBx® cells, preferably chicken EB 14 cells, have an increased ADCC activity compared to the same antibody produced in hybridoma and CHO cells. This is achieved by providing the antibodies of interest with the avian EBx® glycosylation pattern, more preferably with chicken EBx® glycosylation pattern or duck EBx® glycosylation pattern. In particular, the transfected avian EBx® cells of the invention allow to express a large proportion of antibodies or fragment thereof, carrying a common N-linked oligosaccharide structure of a biantennary-type that comprises long chains with terminal GlcNac that are highly galactosylated and non-fucosylated and which confer strong ADCC activity to antibodies. Among a recombinant antibody population produced in EBx® cells, the proportion of non-fucosylated antibodies represent at least 20%, more preferably at least 35%, and more preferably at least 45% of the antibodies or higher. EBx® cells protein production platform is therefore useful to increase Fc-mediated cellular cytotoxicity against undesirable cells mediated by an immunoglobulin or a fragment thereof or by any biological molecule carrying a Fc region of an immunoglobulin region, or an equivalent to the Fc region of an immunoglobulin.

As used herein, the term Fc-mediated cellular cytotoxicity includes antibody-dependent cellular cytotoxicity and cellular cytotoxicity mediated by a soluble Fc-fusion protein containing a human Fc-region. It is an immune mechanism leading to the lysis of "antibody-targeted cells" by "human immune effector cells". "Human immune effector cells" are a population of leukocytes that display Fc receptors on their surface through which they bind to the Fc-region of antibodies or of Fc-fusion proteins and perform effector functions. Such a population may include, but is not limited to, peripheral blood mononuclear cells (PBMC) and/or natural killer (NK) cells. The antibody-targeted cells are cells bound by the antibodies or Fc-fusion proteins. As used herein, the term increased Fc-mediated cellular cytotoxicity is defined as either an increase in the number of "antibody-targeted cells" that are lysed in a given time, at a given concentration of antibody, or of Fc-fusion protein, in the medium surrounding the target cells, by the mechanism of Fc- mediated cellular cytotoxicity defined above, and/or a reduction in the concentration of antibody, or of Fc-fusion protein, in the medium surrounding the target cells, required to achieve the lysis of a given number of "antibody-targeted cells", in a given time, by the mechanism of Fc-mediated cellular cytotoxicity. The increase in Fc-mediated cellular cytotoxicity is relative to the cellular cytotoxicity mediated by the same antibody, or Fc-fusion protein, produced by the other type of host cells such as for example hybridomas, using the same standard production, purification, formulation and storage methods, which are known to those skilled in the art. By antibody having increased antibody dependent cellular cytotoxicity (ADCC) is meant an antibody, as that term is defined herein, having increased ADCC as determined by any suitable method known to those of ordinary skill in the art. One accepted *in vitro* ADCC assay is as follows:
1) the assay uses target cells that are known to express the target antigen recognized by the antigen-binding region of the antibody. The assay uses human peripheral blood mononuclear cells (PBMCs), isolated from blood of a randomly chosen healthy donor, as effector cells;
2) the assay is carried out according to following protocol: i) the PBMCs are isolated using standard density centrifugation procedures and are suspended at 5 x 10⁶ cells/ml in RPMI cell culture medium; ii) the target cells are grown by standard tissue culture methods, harvested from the exponential growth phase with a viability higher than 90%, washed in RPMI cell culture medium, labeled with 100 micro-Curies of ⁵¹Cr, washed twice with cell culture medium, and resuspended in cell culture medium at a density of 10⁵ cells/ml; iii) 100 microliters of the final target cell suspension above are transferred to each well of a 96-well microtiter plate; iv) the antibody is serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above; v) for the maximum release (MR) controls, 3 additional wells in the plate containing the labeled target cells, receive 50 microliters of a 2% (V/V) aqueous solution of non-ionic detergent (Nonidet, Sigma, St. Louis), instead of the antibody solution (point iv above); vi) for the spontaneous release (SR) controls, 3 additional wells in the plate containing the labeled target cells, receive 50 microliters of RPMI cell culture medium instead of the antibody solution (point iv above); vii) the 96-well microtiter plate is then centrifuged at 50 x g for 1 minute and incubated for 1 hour at 4°C; viii) 50 microliters of the PBMC suspension (point i above) are added to each well to yield an effector: target cell ratio of 25:1 and the plates are placed in an incubator under 5% CO₂ atmosphere at 37°C for 4 hours; ix) the cell-free supernatant from each well is harvested and the experimentally released radioactivity (ER) is quantified using a gamma counter; x) the percentage of specific lysis is calculated for each antibody concentration according to the formula (ER-MR)/ (MR-SR) x 100, where ER is the average radioactivity quantified (see point ix above) for that antibody concentration, MR is the average radioactivity quantified (see point ix above) for the MR controls (see point v above), and SR is the average radioactivity quantified (see point ix above) for the SR controls (see point vi above); 4) "increased ADCC" is defined as either an increase in the maximum percentage of specific lysis observed within the antibody concentration range tested above, and/or a reduction in the concentration of antibody required to achieve one half of the maximum percentage of specific lysis observed within the antibody concentration range tested above. The increase in ADCC is relative to the ADCC, measured with the above assay, mediated by the same antibody, produced by another type of host cells, using the same standard production, purification, formulation and storage methods, which are known to those skilled in the art. For example, antibody of IgG1 subtype produced by the method of the invention have an increased ADCC activity compared to the same antibody produce in hybridoma or CHO cells.

The instant invention relates to the biological product of interest according the invention as a medicament. More specifically, the invention relates to the antibody according the invention as a medicament. The higher cytotoxicity activity of EBx® produced IgG will allow to reduced the amount of antibody administrated to patients and to decreased treatment associated costs.

EBx® glycosylated and non-glycosylated biological products (e.g. viral proteins, antibodies, ...) are useful in medical therapy for preventing and treating numerous human and animal disorders. Non-limiting example of disorders are viral infectious diseases, bacterial infectious diseases, cancers (e.g. Non- Hodgkin lymphoma, multiple myeloma, melanoma, etc.), infectious diseases (AIDS, Herpes, Hepatitis B, Hepatitis C, ...), auto-immune disorders (e.g. multiple sclerosis, graft vs. host disease, psoriasis, juvenile onset diabetes, Sjogrens' disease, thyroid disease, myasthenia gravis, transplant rejection, asthma, etc.), inflammatory disorders (e.g. rheumatoid arthritis, systemic lupis, ...).

The invention therefore provides the use of EBx® glycosylated and non-glycosylated biological products (e.g. antibodies, viral proteins, ...) in the manufacture of a medicament for the prophylactic and therapeutic treatment of any of the aforementioned disorders. Also provided is a method of treating a human being having any such a disorder comprising administering to said individual a prophylactic or therapeutically effective amount of a EBx® glycosylated and non-glycosylated biological products.

The invention also covers the use of a biological product according to the invention for the preparation of a pharmaceutical composition for the prevention or the treatment of human and animal diseases. Such pharmaceutical compositions preferably include, in addition to the biological product, a physiologically acceptable diluent or carrier possibly in admixture with other agents such as for example an antibiotic. Suitable carriers include but are not limited to physiological saline, phosphate buffered saline, phosphate buffered saline glucose and buffered saline. Alternatively, the biological product such as an antibody may be lyophilised (freeze dried) and reconstituted for use when needed by the addition of an aqueous buffered solution as described above. Therefore, the invention provides a pharmaceutical composition comprising the biological product of the invention and a pharmaceutical acceptable carrier.

The dosages of such biological products will vary with the condition being treated and the recipient of the treatment. For an antibody, the dosages are for example in the range 1 to about 100 mg for an adult patient preferably 1-10 mg usually administered daily for a period between 1 and 30 days. Routes of administration are routinely parenteral including intravenous, intramuscular, subcutaneous and intraperitoneal injection or delivery.

The examples below explain the invention in more detail. The following preparations and examples are given to enable those skilled in the art to more clearly understand and to practice the present invention. The present invention, however, is not limited in scope by the exemplified embodiments, which are intended as illustrations of single aspects of the invention only, and methods which are functionally equivalent are within the scope of the invention. Indeed, various modifications of the invention in addition to those described herein will become apparent to those skilled in the art from the foregoing description and accompanying drawings. Such modifications are intended to fall within the scope of the appended claims. For the remainder of the description, reference will be made to the legend to the figures below.

### FIGURES

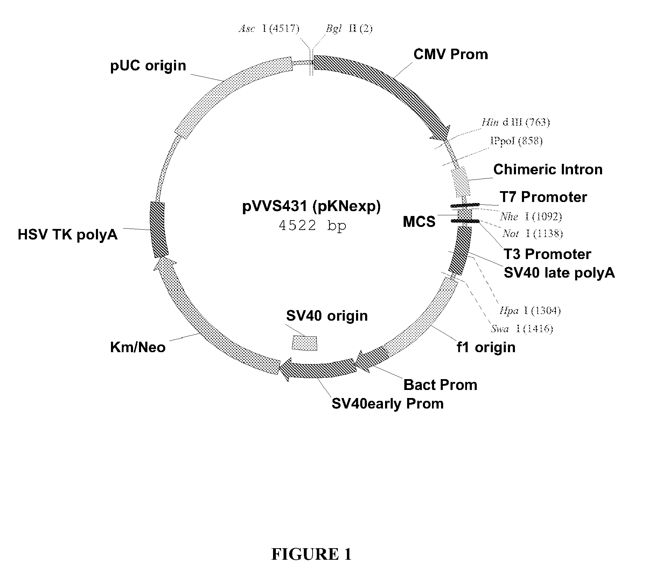

### FIGURE 1: Parental empty expression vector: pVVS431 (pKNexp)

VIVALIS vector backbone comprises a single resistance cassette to allow plasmid amplification in *E.coli* and clone selection after transfection of expression vectors in avian cells. The *nptII* gene encodes the neomycin phospotransferase protein and gives to the organism resistance to Kanamycin in prokaryotes and to Neomycin in eukaryotes. Transcription is driven by both prokaryotic and eukaryotic promoters cloned in tandem upstream *nptII* gene. cDNAs encoding proteins of interest is cloned within a MCS (multiple cloning site) located in an empty expression cassette harbouring an easely exangeable promoter (i.e CMV promoter...), an intron and a polyA region.

### FIGURES 2A and 2B:

Human-mouse chimeric Mab IgG1 (kappa light and heavy chains) was kindly obtained from MAT-Biopharma (Evry, France).

### Figure 2A: Vector bearing RSV promoter driving IgG1 light chain expression : pVVS452 (pRSV-IgG1-L)

### Figure 2B: Vector bearing RSV promoter driving IgG1 heavy chain expression: pVVS450 (pRSV-IgG1-H)

### FIGURES 3A and 3B: Dual vectors expressing both IgG1 light and heavy chains under control of RSV promoter

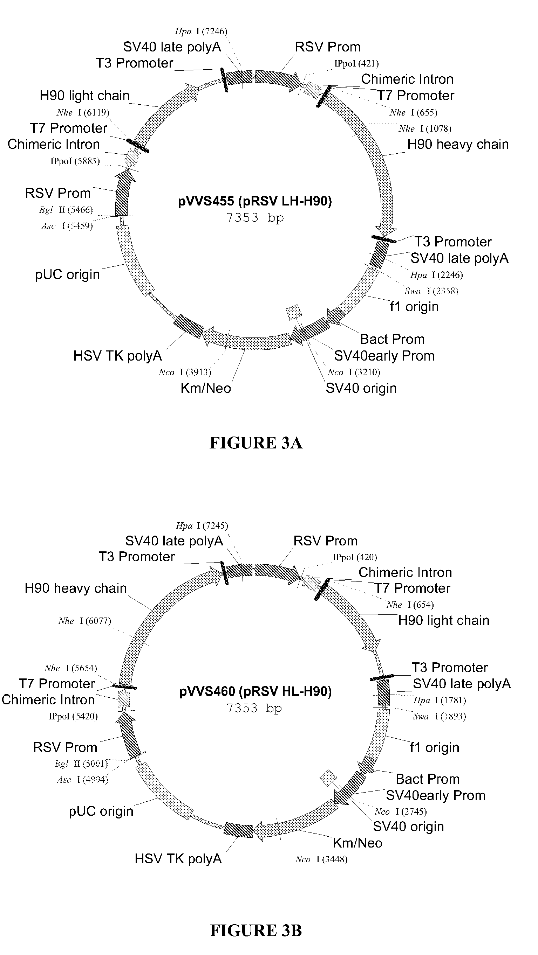

### Figure 3A: pVVS455 (pRSV-LH-IgG1)

### Figure 3B: pVVS460 (pRSV-HL-IgG1)

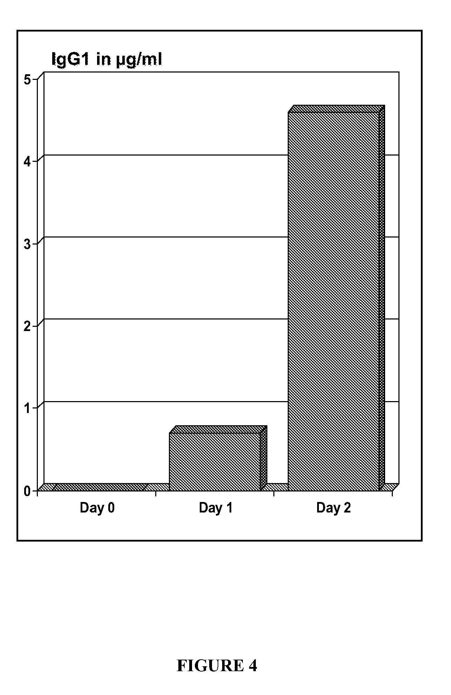

### FIGURE 4: Transient expression of IgG1 in duck EBx® cells in serum free medium

Duck EBx cells were transiently expressed in duck EBx® cells. IgG1 concentration was determined by ELISA 24H and 48H post-transfection. The antibody concentration reached approx. 4.5 ug/ml in cell culture supernatant.

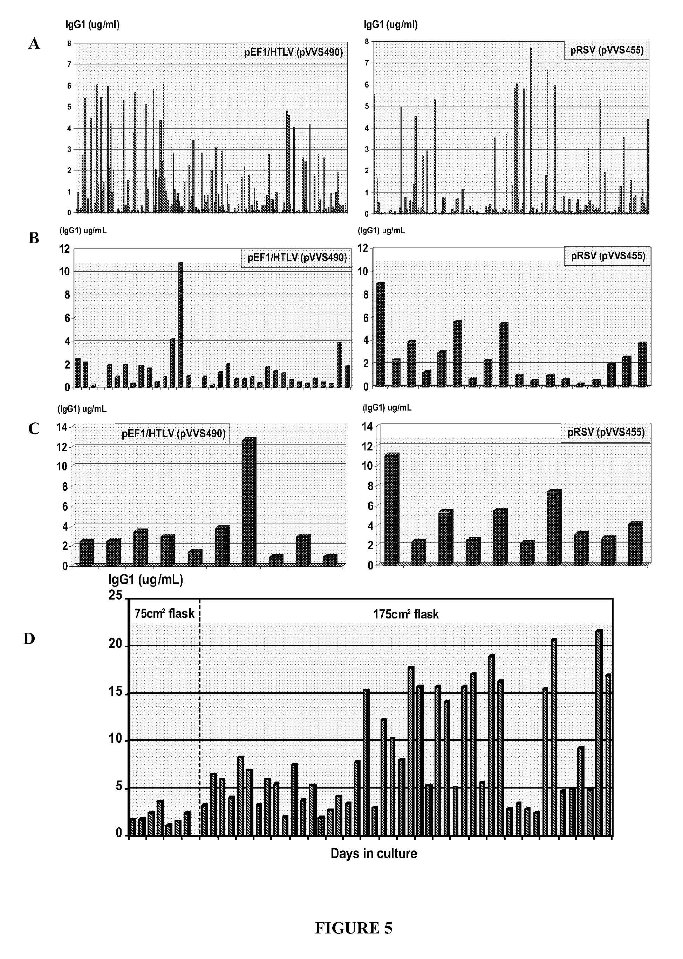

**FIGURE 5****: Stable transfection, clones isolation and screening for IgG1 production Panel A:** Chicken EB14 cells were transfected either with IgG1 expression vector with EF1/HTLV promoter (left) or with RSV promoter (right). 200 resistant colonies from chicken EB14 transfections were picked and seeded in Excell 63066 medium (SAFC Biosciences), 0.25 mg/ml Geneticin. After 5 days of growth post seeding, an ELISA assay was performed to analyse IgG1 production from each picked colony. **Panel B:** Only best producers were amplified in 24 well plates. Panel C: Only best producers were amplified and passed in 6 well plates. Panel D: Finally, only best producers have been grown up to 175cm²-flask. This panel D shows an example of IgG1 productivity, monitored by ELISA assay, during routine culture of one chicken EB14 clone (EF1/HTLV promoter).

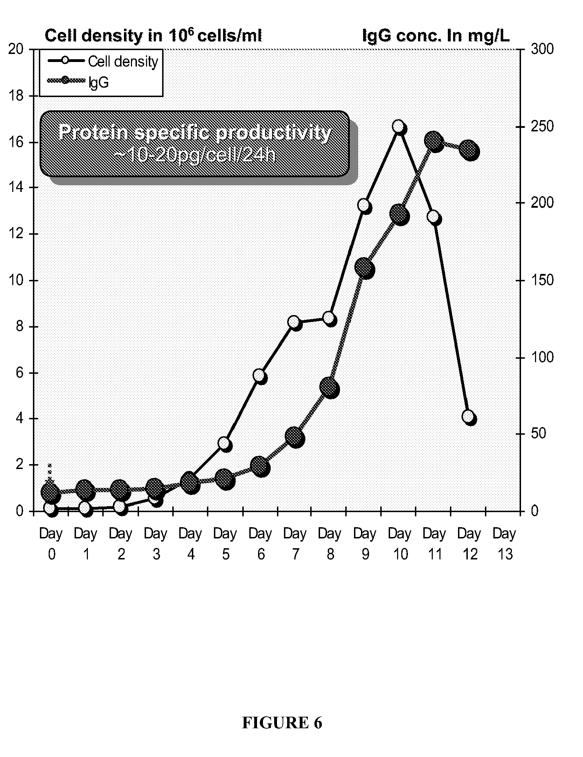

### FIGURE 6: IgG1 antibody produced in chicken EB14 cells in stirred-tank bioreactor

Stable transfected chicken EB14 clone expressing IgG1 (EF1/HTLV promoter) was adapted to growth in suspension in serum free ExCell medium (SAFC BioSciences). A 3 liters stirred-tank bioreactor (Applikon) was seeded with 0.4 million cell/ml. Then a batch culture was performed; cells were allowed to grow during 13 days. EB 14 cells reached a maximum cell density of 16 millions cells/ml at day 10. IgG1 concentration in cell culture medium was monitored by ELISA assay. At day 12, the maximum IgG1 concentration reached 0.25 g/l. The protein specific productivity is around 10-20 pg/cell/24h.

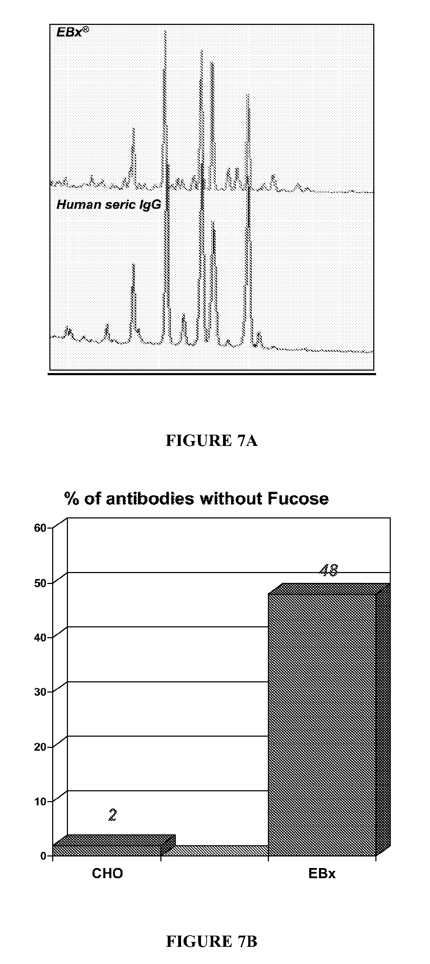

### FIGURES 7A and 7B: Glycosylation profile of IgG1 antibody produced in chicken EBx cells

### Figure 7A: Capillary electrophoresis analysis

The glycosylation profile of IgG1 produced in chicken EB14 cells is similar to the one of human seric IgG molecules.

### Figure 7B: Mass spectroscopy analysis

The percentage of fucose-less IgG1 antibody reached 48% of antibody population produced in chicken EB 14 cells versus only 2% of antibody produced in CHO cells (data from literature).

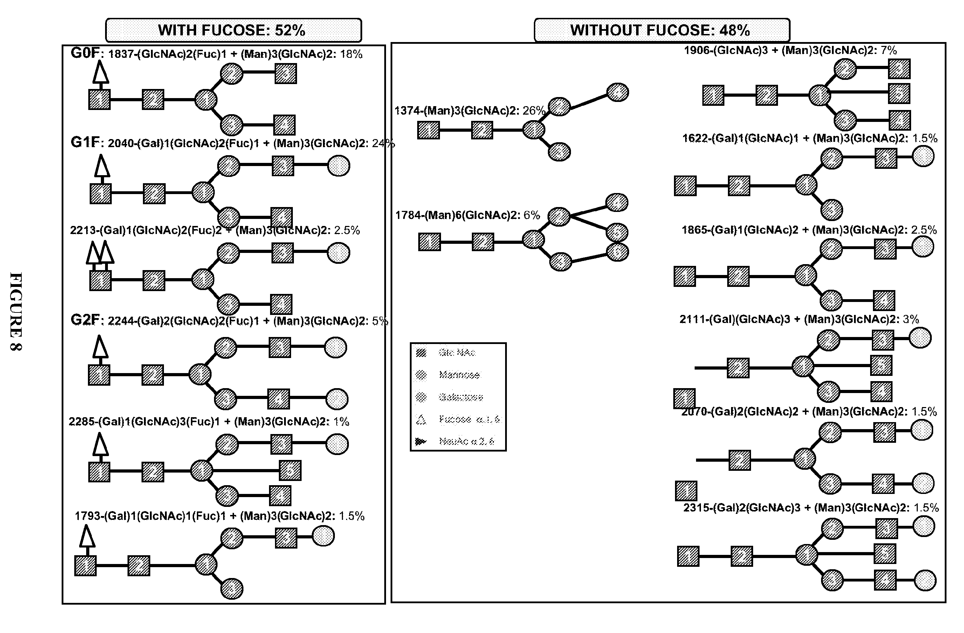

### FIGURE 8: Detailed glycosylation profile of IgG1 antibody produced in chicken EBx cells

The glycosylation profile of an IgG1 produced in one stably transfected clone of chicken EB14 cells were analysed by Maldi-Toff mass spectrum analysis. The structure of N-oligosaccharide attached to the CH2 domain of each IgG1 heavy chain at residue Asn 297 were analyzed . Most of IgG1 antibodies produced in this chicken EB 14 clone has a common N-linked oligosaccharide structure of a bi-antennary type that comprises long chains with terminal GlcNac, that are galactosylated. An important part (48%) of IgG1 antibodies population is not fucosylated. Some antibodies population have a N-linked oligosaccharide structure of a bi-antennary type with bisecting GlcNac.

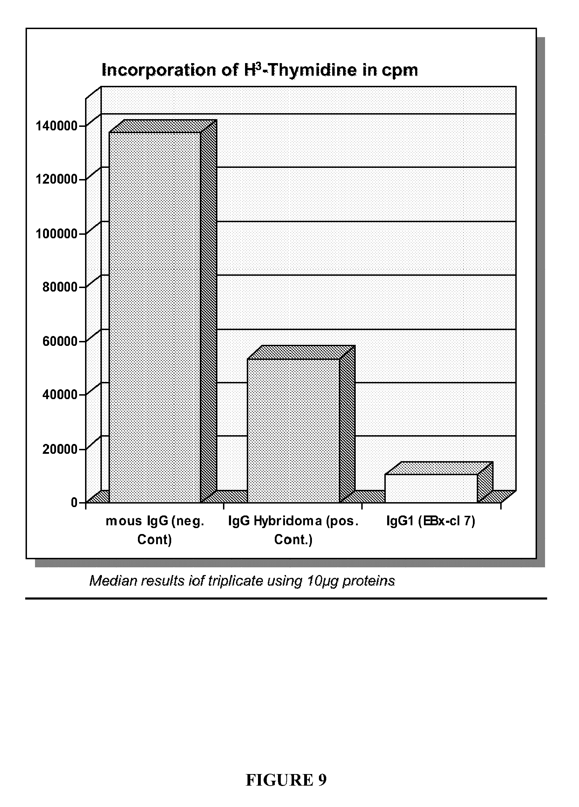

### FIGURE 9: Inhibition of proliferation of tumor cells with IgG1 antibody produced in chicken EBx cells

An assay was developed to measure cellular proliferation inhibition activity of an IgG1 immunoglobulin either produced in chicken EB14 cells or in an hybridoma. The IgG1 antibody is directed against a CD marker expressed on human tumor cells surface. Tumor cells were grown in presence of tritiated thymidine and were incubated with monocytes or natural killer T cells purified from a normal patient, in presence of IgG1 immunoglobulin. The assay measures amount of tritiated thymidine incorporated in proliferating tumor cells as a consequence of IgG1 mediated tumor cell lysis by Natural Killer cells. After 4 days in culture, low amount of tritiated thymidine were incorporated in tumor cells treated NK cells and with IgG1 antibody produced in chicken EB14 cells indicating that tumor cells did not proliferate. In the opposite, a higher level oh H3-thymidine was observed in tumor cells treated with NK or monocytes cells and with IgG1 antibody produced in hybridoma indicating that tumor cells proliferated. IgG1 produced in chicken EB14 cells display a better cell anti-proliferative activity that the same antibody produced in hybridoma.

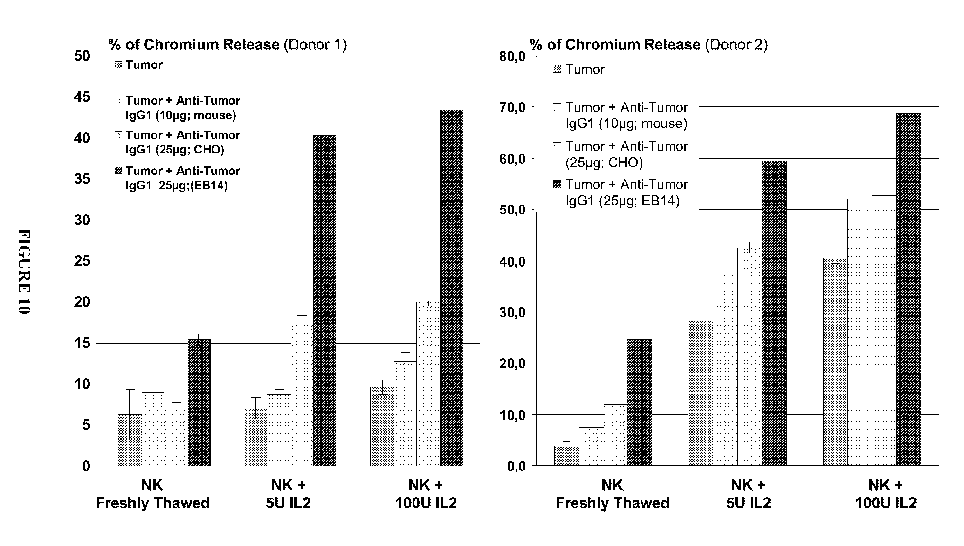

### FIGURE 10: Anti-tumor Cytotoxic Response of IgG1 antibody produced in chicken EB14 vs CHO cells

A standard ADCC assay were performed using Natural Killer cells from two healthy donors. NK cells were either cultured without Interleukine 2 (IL-2) or with 5 or 100 units of IL-2. Tumor cells previously cultured with radioactive chromium were incubated with IgG1 immunoglobulin and NK cells. The ADDC activity was evaluated as a % of chromium release in the medium.
Tumor: Negative control
Tumor + Anti-Tumor IgG1 from hybridoma (non chimerized) : Positive control
Tumor + Anti-Tumor IgG1 produced in CHO
Tumor + Anti-Tumor IgG1 produced in chicken EB14 cells
IgG1 antibody produced in chicken EB14 cells display a higher ADCC activity as compared to the same IgG1 antibody produced in CHO cells.

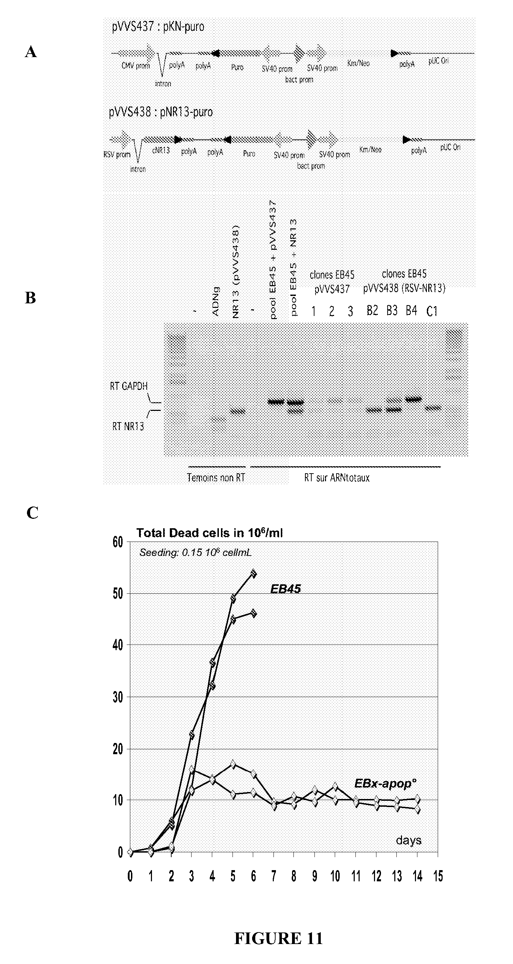

### FIGURE 11: Growth analysis of adherent chicken EBx® cells expressing NR13 anti-apoptotic gene

**PANEL A:** Description of pVVS437 and pVVS438 vectors. pVVS437 harbors only the puromycine resistance gene. PVVS438 allows the expression of puromycine resistance and of the chicken anti-apoptotic gene NR13. Adherent chicken EBx® cells (EB45) have been transfected with pVVS437 or pVVS438 vectors and selection has been performed on these cells.

**Panel B**: Clones have been pooled in population or isolated and RT-PCR has been performed using total RNA isolated from these populations or clones to detect the expression of NR13 gene. This panel shows that clones B2, B3 and C1 express the anti-apoptotic gene NR13.

**Panel C:** Growth analysis of adherent chicken EBx® cells (EB45) stably transfected with NR13 anti-apoptotic gene. Stably transfected EBx® cells that express NR13 protein were cultured in adherence in 100 mm dishes. EBx® cells that do not expressed NR13 protein do not survive into culture and most of them are dead after 6 to 7 days in culture. Only a small and stable proportion of EBx® expressing NR13 protein are dead in the culture, the majority of NR13 EBx® cells is staying alive for a longer period of time in culture.

### EXAMPLES

### EXAMPLE 1: chicken EBv13 cell line from SPF chicken strain VALO

### 1.1 - RAW MATERIAL

### Eggs

Specific Pathogen Free (SPF) strain called Valo. The valo strain is a white Leghorn strain produced and delivered by Lohmann from Germany. Those SPF chicken eggs, supplied with a certificate of analysis, are tested for: CAV, Avian adenoviruses (group 1, serotypes 1-12 and group 3), EDS, Avian Encephalomyelitis Virus, Avian Leukosis Viruses/RSV (including Serotype ALV-J), Avian Nephritis Virus, Avian Reoviruses, Fowlpox Virus, Infectious Bronchitis Virus, Infectious Bursitis Virus (IBDV), Infectious Laryngo Tracheitis Virus, Influenzavirus Typ A, Marek's Disease Virus, Mycoplasmosis (Mg + Ms), Mycobacterium avium, Newcastle Disease Virus, Reticuloendotheliosis Virus, Salmonella pullorum, Other Salmonella Infections, Avian Rhinotracheitis Virus (ART), Hemophilus paragallinarum. Valo chicken eggs were only submitted to a disinfection with the decontaminant to avoid any risk of contamination linked to the manipulation of eggs during the transport.

### Feeder cells

In the first step of the process of establishment of EBv13, cells from murine origin (STO cells) were used as feeder layer to maintain the pluripotency of chicken stem cells. Those feeder cells are mitotically inactivated by gamma irradiation (45 to 55 Grays) before seeding on plastic. This dose of irradiation is a sub-lethal dose that induces a definitive arrest of the cell cycle but still permits the production of growth factors and extracellular matrix, necessary for the promotion of the cell growth of non differentiated cells.

The STO cell line was derived by A. Bernstein, Ontario Cancer Institute, Toronto, Canada from a continuous line of SIM (Sandos Inbred Mice) mouse embryonic fibroblasts and it was supplied by the American Type Culture Collection (ATCC) (STO Product number: CRL-1503, Batch number 1198713). Fresh feeder layers were prepared twice a week, in general on monday and thursday. Exponentially cells were dissociated and counted. A part of cells were seeded for maintenance of viable cultures and another part was irradiated. For irradiation, we prepared a cell suspension at 10x10⁶ cells/mL in tubes. Cells were exposed to a 45 to 55 grey dose and were seeded on plastic. After seeding, dishes or plates coated with inactivated feeder cells were used during a maximum of 5 days.

### Medium

DMEM- HamF12 (Cambrex, Cat n° BE04-687)
Optipro medium (Invitrogen, Cat n° 12309)
EX-CELL*^{™}* 65195, 60947 and 65319 (SAFC, customized medium)

### Additives

Glutamine (Cambrex, Cat n° BE17-605E)
Pencillin/streptomycin (Cambrex, Cat n° BE17-602E))
Non essential Amino Acids (Cambrex, Cat n° BE13-114E)
Sodium pyruvate (Cambrex, Cat n°BE13-115)
Vitamines (Cambrex, Cat n° 13-607C)
Beta Mercapto Ethanol (Sigma, Cat n° M7522)

### Buffer and fixators

PBS 1X (Cambrex, Cat n° BE17-516F)
Paraformaldehyde 4 % (Sigma, Cat n° P6148)
KC1 5,6 % (Sigma, Cat n° P9333)
Methanol/ Acetic acid (3/1): Methanol (Merck, Cat n° K34497209 ; Acetic acid Sigma Cat n°A6283)
Colcemid, Karyomax (Gibco, Cat n° 15212-046)

### Cryoprotective agent

Dimethyl Sulfoxyde (DMSO) (Sigma, Cat n° D2650)

### Factors

Two different recombinant factors were used:
□ Recombinant Human Ciliary Neurotrophic Factor (CNTF) (Peprotech Inc, Cat n° 450-13)
□ Recombinant Human Insulin Like Factor I (IGF1) (Peprotech Inc, Cat n° 100-11) The two factors were produced in E. Coli bacteria.

### Fetal Bovine Serum

### Non irradiated Fetal Bovin Serum (FBS) (JRH, Cat n° 12103)

The non irradiated serum used in the program was collected and produced in United States. Animals used for collection were USDA inspected and acceptable for slaughter. It was added in the medium during avian stem cells culture. This batch was not submitted to irradiation to avoid the destruction of critical proteins or components identified as essential for the maintenance of stem cells in culture.

### Irradiated serum (JRH, Cat n° 12107)

The irradiated batch used in this program was also collected in United States. This irradiated batch was added as supplement in the DMEM medium used for the culture of STO or FED cells (feeder cells). Those cells do not require as stem cells a specific quality of serum for growth and maintenance in culture. To minimize high concentration of serum in the medium we have adapted the STO cells to grow in presence of 4 % of FBS only.

### Dissociating agents:

### • Pronase (Roche, Cat n° 165 921)

Pronase is a recombinant protease manufactured by Roche Diagnostics, Germany, used for the dissociation of adherent avian stem cells.

### • Trypsin EDTA (Cambrex, cat n° BE17-161E)

Trypsin is used for the dissociation of STO or FED cells and at late passages for the dissociation of avian cells adapted to Serum Free Medium. This enzyme of porcine origin is manufactured aseptically according to cGMP referential conditions by a validated sterile filtration method and tested according to current E.P. The raw material, irradiated prior to formulation, is tested for porcine parvovirus in strict compliance with 9/CFR 113.53.

### • Non enzymatic cell dissociation solution (Sigma, Cat n° C5914)

This agent of dissociation is a ready to use formulation used to gently detach cells from the growing surface of the culture vessel. The formula contains no protein, and allows dislodging of cells without use of enzymes. Cellular proteins are preserved making possible immunochemical studies that are dependent upon the recognition of cell surface proteins. This enzyme was used to detach cell before FACS analysis of biological markers like EMA-1 (Epithelial Membrane Antigen 1) and SSEA1 (Stage Specific Embryonic antigen-1).

### 1.2 - PROCESS OF ESTABLISHMENT OF EBv13 CELL LINE

Eggs are opened, the yolk were separated from the albumen during the opening. The embryos were removed from the yolk either directly with the aid of a Pasteur pipette, or with the aid of a small absorbent filter paper (Whatmann 3M paper), cut out beforehand in the form of a perforated ring with the aid of a punch. The diameter of the perforation were about 5 mm. These small rings were sterilized using dry heat for about 30 minutes in an oven. This small paper ring is deposited on the surface of the yolk and centered on the embryo which is thus surrounded by the paper ring. The latter is then cut out with the aid of small pairs of scissors and the whole removed is placed in a Petri dish, filled with PBS or with a physiological saline. The embryo thus carried away by the ring were cleaned of the excess yolk in the medium and the embryonic disk, thus free of the excess vitellin, is collected with a Pasteur pipette.

The chicken Valo embryos were placed in a tube containing physiological medium (1X PBS, Tris Glucose, medium, and the like). The Valo embryos were then mechanically dissociated and inoculated on a layer of feeder STO cells into complete culture medium at 39°C. The feeder cells were seeded in flask at around 2.7x10⁴ cell/cm². The complete culture medium is composed of basal commercial medium DMEM-Ham F12 supplemented with 10 % fetal calf serum, with IGF1 and CNTF at a final concentration of 1ng/ml, and with 1 % non-essential amino acids, with 1 % of mixture of vitamins of commercial origin, with sodium pyruvate at a final concentration of 1 mM, with beta-mercapto-ethanol at a final concentration of 0.2 mM, glutamine at a final concentration of 2.9 mM, with an initial mixture of antibiotics containing penicillin at a final concentration of 100 U/ml and streptomycin at a final concentration of 100 µg/ml. Rapidly after the first passages of the cells, the mixture of antibiotics is no longer added to the medium. The expression rapidly is understood to mean after the first 3 to 5 passages in general.

When the avian ES cells from chicken Valo embryos is passaged from a culture flask to another, the seeding of culture flasks was performed with around between 7 x 10⁴/cm² to 8 x 10⁴/cm² of avian ES cells in the complete culture medium. Preferably, the seeding is made with around 7.3 x 10⁴/cm² (4 x 10⁶ cells/55cm² or 4 x 10⁶ cells/100 mm dish). The avian cells, preferably the avian embryonic cells of step a) are cultured during several passages in the complete medium. At passage 15, the complete medium was depleted in growth factors IGF1 and CNTF. The depletion is made directly in one step, from one passage to another. The embryonic stem cells, preferably the avian embryonic cells are cultured during several passages in the complete medium without IGF1 and CNTF growth factors.

Then depletion of feeder cells were performed after the depletion of growth factors IGF1 and CNTF by a progressive decreasing of feeder cells concentration over several passages. Practically, the same concentration of the feeder cells were used for 2 to 4 passages, then a lower concentration of the feeder cells were used for an additional 2 to 4 passages, and so on. The flask were originally seeded with around 2.7 x10⁴ feeder cells/cm², then around 2.2 x 10⁴ feeder cells/cm², then around 1.8 x 10⁴ feeder cells/cm², then around 1.4 x 10⁴ feeder cells/cm², then around 1.1 x 10⁴ feeder cells/cm², then around 0.9 x 10⁴ feeder cells/cm², then around 0.5 x 10⁴ feeder cells/cm². Then the flask were seeded with 6.5 x 10⁴ avian cells/cm² to 7.5 x 10⁴ avian cells/cm² and without feeder cells. The depletion of feeder cells started at around passage 21 and ended at around passage 65. During the depletion of feeder cells, the chicken Valo ES cells were seeded in culture flask at a lower concentration than in step a), about around 4 x 10⁴ cell/cm² to 5 x 10⁴ cell/cm². In the hypothesis that Valo ES cells were not in good shape following a decrease of feeder cells concentration in the flask, then the avian cells are cultured for additional passages with the same feeder cells concentration before to pursue the feeder cells depletion.

The serum depletion were performed after the growth factor and the feeder cells depletion. At the beginning of serum depletion, the culture medium were composed of basal commercial medium DMEM-HamF12 supplemented with 10 % fetal calf serum and with 1 % non-essential amino acids, with 1 % of mixture of vitamins of commercial origin, with sodium pyruvate at a final concentration of 1 mM, with beta-mercaptoethanol at a final concentration of 0.2 mM, glutamine at a final concentration of 2.9 mM. The chicken Valo cells were adapted to the growth in a serum free medium culture in a two steps process: first, the chicken Valo cells were rapidly adapted to a culture medium composed of commercial serum free medium (SFM), preferably ExCell 60947 (SAFC Biosciences) supplemented with 10 % fetal calf serum and with 1 % non-essential amino acids, with 1 % of mixture of vitamins of commercial origin, with sodium pyruvate at a final concentration of 1 mM, with beta-mercaptoethanol at a final concentration of 0.2 mM, glutamine at a final concentration of 2.9 mM. Once this rapid adaptation to a new medium (DMEM-HamF12 to Excell 60947) was performed, a second step is performed consisting of a slow adaptation to decreasing concentration of animal serum in the SFM medium were initiated. Serum depletion was performed by a progressive decreasing starting from 10 % serum, then 7.5 %, then 5 %, then 2.5 %, then 1.25 %, then 0,75 % of serum concentration in SFM cell culture medium to finally reach 0 % serum in SFM cell culture medium. Serum depletion started at passage 103 and ended at passage 135.

At the end of the process of deprivation of serum when the remaining concentration of serum in SFM medium was either 0.75 % or 0 %, the adaptation of anchorage-dependent EBv13 cells to suspension culture started. Among the several attempts performed to isolate anchorage-independent EBv13 isolates, 62.5 % of the attempts were successful and allow to get different isolates of suspension EBv13 cells. One isolate of EBv13 cells were selected according to the population doubling time (around 18h), the optimal cell concentration into flask culture (around 4 million cell/ml), the cell viability, the cell culture homogeneity (presence and size of cells clumps) and the easiness to manipulate the cells.

At the end of serum depletion, anchorage dependent chicken Valo cells, named EBv13 were able to grow in absence of grow factors, in absence of feeder cells, in serum free medium. EBv13 Cells were then adapted to growth at 37°C, by progressively decreasing cell culture temperature of 0.5°C/day.

### EXAMPLE 2: Duck EBx cell line EB66

### 2.1 - RAW MATERIAL

### Duck Eggs

Duck eggs from Peking strains GL30 were obtained from GRIMAUD FRERES SELECTION (La Corbière, Roussay France). The parent ducks were vaccinated against *Escherichia Coli* (Autogenous vaccine Coli 01 & 02), *Pasteurella multocida* (Landavax), Duck viral hepatitis (Hepatovax), *Erysipelothrix rhusiopathiae* (Ruvax), Avian metapneumovirus (Nemovac), *Salmonella typhimurium & Enteridis* (Autogenous vaccine), *Riemerella antipestifer* (Autovaccine Riemerella), Avian metapneumovirus (Nobilis RTV inactive) and *Erysipelothrix rhusiopathiae* (Ruvax). After receipt, fertilized Peking duck eggs were submitted to a disinfection in an hypochloryde bath followed by a decontamination with Fermacidal (Thermo) to avoid any risk of contamination linked to dusts attached on the shell.

### Feeder cells

In the first step of the process, cells from murine origin (STO cells) were used as feeder layer to maintain the pluripotency of duck stem cells. Those feeder cells are mitotically inactivated by gamma irradiation (45 to 55 Grays) before seeding on plastic. This dose of irradiation is a sub-lethal dose that induces a definitive arrest of the cell cycle but still permits the production of growth factors and extracellular matrix, necessary for the promotion of the cell growth of non differentiated cells. The STO cell line was derived by A. Bernstein, Ontario Cancer Institute, Toronto, Canada from a continuous line of SIM (Sandos Inbred Mice) mouse embryonic fibroblasts and it was supplied by the American Type Culture Collection (ATCC) (STO Product number: CRL-1503, Batch number 1198713). Fresh feeder layers were prepared twice a week. Exponentially cells were dissociated and counted. A part of cells were seeded for maintenance of viable cultures and another part was irradiated. For irradiation, we prepared a cell suspension at 10x10⁶ cells/mL in tubes. Cells were exposed to a 45 to 55 grey dose and were seeded on plastic. After seeding, dishes or plates coated with inactivated feeder cells were used during a maximum of 5 days.

### Medium

Medium EX-CELL*^{™}* 65788, 65319, 63066 and 66444 (SAFC, customized medium)
Medium GTM-3 (Sigma, Cat n° G9916)
DMEM- HamF12 (Cambrex, Cat n° BE04-687)
DMEM (Cambrex, Cat n° BE 12-614F)

### Additives

Glutamine (Cambrex, Cat n° BE17-605E)
Pencillin/streptomycin (Cambrex, Cat n° BE17-602E))
Non essential Amino Acids (Cambrex, Cat n° BE13-114E)
Sodium pyruvate (Cambrex, Cat n°BE13-115)
Vitamines (Cambrex, Cat n° 13-607C)
Beta Mercapto Ethanol (Sigma, Cat n° M7522)
Yeastolate (SAFC, Cat n° 58902C)

### Buffer and fixators

PBS 1X (Cambrex, Cat n° BE17-516F)

### Cryoprotective agent

Dimethyl Sulfoxyde (DMSO) (Sigma, Cat n° D2650)

### Factors

Two different recombinant factors were used:
□ Recombinant Human Ciliary Neurotrophic Factor (CNTF) (Peprotech Inc, Cat n° 450-13)
□ Recombinant Human Insulin Like Factor I (IGF1) (Peprotech Inc, Cat n° 100-11) Those 2 factors are produced in E. Coli bacteria.

### Fetal Bovine Serum

### Non irradiated Fetal Bovin Serum (FBS) (JRH, Cat n° 12003)

The non irradiated serum used in the program was collected and produced in Australia. Animals used for collection were USDA inspected and acceptable for slaughter. It was added in the medium during avian stem cells culture. This batch was not submitted to irradiation to avoid the destruction of critical proteins or components identified as essential for the maintenance of stem cells in culture.

### Irradiated serum (JRH, Cat n° 12107)

The irradiated batch used in this program was collected in United States. This irradiated batch was added as supplement in the DMEM medium used for the culture of STO cells (feeder cells). Those cells do not require as stem cells a specific quality of serum for growth and maintenance in culture. To minimize high concentration of serum in the medium we have adapted the STO cells to grow in presence of 4 % of FBS only.

### Dissociating agents:

### • Pronase (Roche, Cat n° 165 921)

Pronase is a recombinant protease manufactured by Roche Diagnostics, Germany, used for the dissociation of adherent avian stem cells.

### • Trypsine EDTA (Cambrex, cat n° BE17-161E)

Trypsine is used for the dissociation of STO cells and at late passages for the dissociation of avian cells adapted to Serum Free Medium. This enzyme of porcine origin is manufactured aseptically according to cGMP referential conditions by a validated sterile filtration method and tested according to current E.P. The raw material, irradiated prior to formulation, is tested for porcine parvovirus in strict compliance with 9/CFR 113.53.

### • Trypzean (Sigma, cat n° T3449)

Trypzean solution is formulated with a recombinant bovine trypsin, expressed in corn and manufactured by Sigma Aldrich utilizing ProdiGene's proprietary transgenic plant protein expression system. This product is optimized for cell dissociation in both serum free and serum-supplemented adherent cell cultures.

### • Non enzymatic cell dissociation solution (Sigma, Cat n° C5914)

This agent of dissociation is a ready to use formulation used to gently detach cells from the growing surface of the culture vessel. The formula contains no protein, and allows dislodging of cells without use of enzymes. Cellular proteins are preserved making possible immunochemical studies that are dependent upon the recognition of cell surface proteins. This enzyme was used to detach cell before FACS analysis of biological markers like EMA-1 (Epithelial Membrane Antigen 1) and SSEA1 (Stage Specific Embryonic antigen-1).

### 2.2 - PROCESS OF ESTABLISHMENT OF DUCK EBx CELL LINE EB66

Around 360 Fertilized duck eggs were opened, the yolk were separated from the albumen during the opening. The embryos were removed from the yolk with the aid of a small absorbent filter paper (Whatmann 3M paper), cut out beforehand in the form of a perforated ring with the aid of a punch. The diameter of the perforation is about 5 mm. These small rings were sterilized using dry heat for about 30 minutes in an oven. In practice, during the step of embryo collection, a small paper ring is deposited on the surface of the yolk and centered on the embryo which is thus surrounded by the paper ring. The latter is then cut out with the aid of small pairs of scissors and the whole removed is placed in a Petri dish, filled with PBS. The embryo thus carried away by the ring were cleaned of the excess yolk in the medium and the embryonic disk, thus free of the excess vitellin, were collected with a Pasteur pipette.

The duck embryos were placed in 50 ml tubes containing PBS 1X The duck embryos were then mechanically dissociated, washed with PBS, and seeded on an inactivated layer of feeder STO cells into complete culture medium at 39°C, 7,5 % CO₂. The feeder cells were seeded in 6 well plates or dishes at around 2,7 x10⁴ cell/cm². The complete culture medium is composed of serum free medium DMEM-Ham F12 supplemented with 10 % fetal bovine serum, with IGF1, CNTF, at a final concentration of 1ng/ml, and with 1 % non-essential amino acids, with 1 % of mixture of vitamins of commercial origin, with sodium pyruvate at a final concentration of 0,1 mM, with beta-mercapto-ethanol at a final concentration of 0.5 mM, glutamine at a final concentration of 2,1 mM, penicillin at a final concentration of 100 U/ml, streptomycin at a final concentration of 100 µg/ml and yeastolate 1X. Rapidly at the passage 4, the mixture of antibiotics is no longer added to the medium.

The duck ES cells were cultured in the DMEM-Ham F12 medium up to passage 4. After passage 4, the base medium is modified and DMEM-Ham F12 complete medium is replaced by the SFM GTM-3 medium supplemented with 10 % fetal bovine serum, with IGF1, CNTF, at a final concentration of 1ng/ml, with 1 % non-essential amino acids, with 1 % of mixture of vitamins of commercial origin, with sodium pyruvate at a final concentration of 0,1 mM, with beta-mercapto-ethanol at a final concentration of 0.5 mM, glutamine at a final concentration of 2,1 mM and yeastolate 1X. The duck ES cells were further cultured during 14 passages in this new medium of culture, then growth factors deprivation was performed at passage 18. IGF1 and CNTF were simultaneously removed from the medium, thus from passage 19 to passage 24, the medium of culture was GTM-3 medium supplemented with 10 % FBS, with 1 % non-essential amino acids, with 1 % of mixture of vitamins of commercial origin, with sodium pyruvate at a final concentration of 0,1 mM, with beta-mercapto-ethanol at a final concentration of 0.5 mM, glutamine at a final concentration of 2,1 mM and yeastolate 1X.

When the duck ES cells from Peking duck embryos are passaged from a culture dish to another, the seeding of culture dish was performed with around between 7 x 10⁴/cm² to 12 x 10⁴/cm² of duck ES cells in the complete culture medium.

Then, after passage 24, depletion of feeder cells were performed by a progressive decrease of feeder cells concentration over several passages. The dishes were originally seeded with around 2,7 x10⁴ feeder cells/cm², then around 1,8 x 10⁴ feeder cells/cm² between passage 25 and 31, then around 1,4 x10⁴ cells/cm² between passage 32 and 35, then around 1 x 10⁴ feeder cells/cm² between passage 36 and 41, then around 0,7 x 10⁴ feeder cells/cm² between passage 42 and 44, and finally from passage 45 dishes were seeded only with avian cells and without feeder cells. At the end of the feeder depletion, the dishes are seeded with 9 x 10⁴ avian cells/cm² to 12.7 x 10⁴ avian cells/cm². The depletion of feeder cells started at passage 25 and ended at passage 45. During the depletion of feeder cells, the duck ES cells are seeded in culture dishes at a higher concentration than in step a), about around 9 x 10⁴ cell/cm² to 12.7 x 10⁴ cell/cm².

After several passages without feeder cells, growth parameters (Population Doubling Time (PDT) and Density) are studied to confirm cell stability and robustness and to initiate the deprivation of amino acids, vitamins, beta mercaptoethanol, sodium pyruvate and yeastolate. Cells are considered as enough robust to be submitted to such deprivation if, PDT is lower than around 40 hours and cell density higher than around 26x10⁴ cells/cm².

In the case of the present duck EBx® cells development, named EB66, deprivation of vitamins, sodium pyruvate, non essential amino acids and beta mercaptoethanol were initiated at passage 52. All those additives were removed simultaneously from the medium. Thus, between passage 52 and passage 59, the medium of culture is SFM GTM-3 supplemented with glutamine, yeastolate and FBS. Following a short period of adaptation to the new conditions of culture, temperature decreasing was initiated. This decrease was performed progressively between passage 60 and passage 67. After passage 67 cells were able to grow at 37°C. After passage 67, the base medium GTM-3 was replaced by a new SFM base medium called Excell 65788. So, after passage 67 the culture medium was Excell 65788 supplemented with 10 % FBS, 2,5 mM glutamine and 1X yeastolate. At passage 80, 4x10⁶ cells were transferred in a Ultra Low Attachment (ULA) dish maintained under constant agitation to initiate anchorage-independent cells growth. To promote the growth as suspension, the base medium was modified and percentage of serum was decreased from 10 % to 5 % for the seeding in the ULA dish. Thus from passage 80 to passage 85 the medium of culture was SFM GTM-3 supplemented with 5 % FBS, 2.5mM glutamine and 1X yeastolate. Slow decrease of FBS was initiated on EB66 cell suspension after passage 85. Serum depletion was performed by a progressive decreasing starting from 2.5 % serum, then 1,5 % of serum concentration in SFM cell culture medium to finally reach 0 % serum in SFM cell culture medium. Serum depletion started at passage 86 and ended at passage 94. At the end of serum depletion, anchorage independent dEB66 cells were able to grow at 37°C in absence of grow factors, in absence of feeder cells, in serum free medium.

After the obtaining of EB66 duck cells that are able to grow at 37°C in the SFM GTM-3 supplemented by 2,5 mM glutamine, some further adaptation to SFM media were made by dilution or progressive adaptation in new SFM formulations as Excell 63066, Excell 66444, Excell CHO ACF for example.

The subcloning of suspension duck EB66 cell could also realized in presence or absence of yeastolate

### EXAMPLE 3: Duck EBx cell line EB26

### 3.1 - RAW MATERIAL

### Duck Eggs, Feeder cells, additives, Buffers and Fixators, Cryopreservative agents, Fetal Calf Serum & dissociating agents (Idem as Example 3).

Duck eggs from Peking strains GL30 were used.

### Medium

Medium EX-CELL 65319, 63066 and 66444 (SAFC, customized medium)
Medium GTM-3 (Sigma, Cat n° G9916)
DMEM (Cambrex, Cat n° BE 12-614F)

### Factors

Six different recombinant factors were used:
□ Recombinant Human Ciliary Neurotrophic Factor (CNTF) (Peprotech Inc, Cat n° 450-13)
□ Recombinant Human Insulin Like Factor I (IGF1) (Peprotech Inc, Cat n° 100-11)
□ Recombinant Human Interleukin 6 (IL6) (Peprotech Inc, Cat n° 200-06)
□ Recombinant Human soluble Interleukin 6 receptor (sIL6r) (Peprotech Inc, Cat n° 200-06 R)
□ Recombinant Human Stem Cell Factor (SCF) (Peprotech Inc, Cat n° 300-07)
□ Recombinant Human basic Fibroblast Growth Factor (bFGF) (Peprotech Inc, Cat n° 100-18B)

All those factors, excepted IL6r, are produced in E. Coli bacteria. Soluble IL6r is expressed in transfected HEK293 cells.

### 3.2 - PROCESS OF ESTABLISHMENT OF DUCK EBx CELL LINE EB26

The duck embryos were collected as previously described with EB66. The duck embryos were placed in 50 mL tubes containing PBS 1X. The duck embryos were then mechanically dissociated, washed in PBS, and seeded on an inactivated layer of feeder STO cells into complete culture medium at 39°C, 7,5 % CO₂. The feeder cells were seeded in 6 well plates or dishes at around 2,7 x10⁴ cell/cm². The complete culture medium is composed of serum free medium GTM-3 supplemented with 5 % fetal bovine serum, with IGF1, CNTF, Il-6, Il-6R, SCF and FGF at a final concentration of 1ng/ml, and with 1 % non-essential amino acids, with 1 % of mixture of vitamins of commercial origin, with sodium pyruvate at a final concentration of 0,1 mM, with beta-mercapto-ethanol at a final concentration of 0.5 mM, glutamine at a final concentration of 2,1 mM, penicillin at a final concentration of 100 U/ml, streptomycin at a final concentration of 100 µg/ml and yeastolate 1X. Rapidly after the first passages of the cells, the mixture of antibiotics is no longer added to the medium. The expression rapidly is understood to mean after the first 3 to 9 passages in general. The duck ES cells were cultured in the complete medium up to passage 9. After passage 9, the complete medium is partially depleted in factors. Thus, between passage 10 and 13, SCF, IL6, IL6r and bFGF were removed for the medium and only recombinant IGF1 and CNTF were maintained at a concentration of 1 ng/ml. A simultaneous decease of concentration of IGF1 and CNTF is secondly performed between passage 13 and 16 to finally obtain cells able to grow without recombinant factors at passage 17. The factor depletion were made by a progressive adaptation to lower concentrations of factors. When the duck ES cells from Pekin duck embryos were passaged from a culture dish to another, the seeding of culture dish was performed with around between 7 x 10⁴/cm² to 12 x 10⁴/cm² of duck ES cells in the complete culture medium. Preferably, the seeding is made with around 7.3 x 10⁴/cm² (4 x 10⁶ cells/55cm² or 4 x 10⁶ cells/100 mm dish). After depletion of recombinant factors, a decrease of yeastolate were performed at passage 23 reaching the final concentration at 0,5X. Then, after passage 31, depletion of feeder cells were performed by a progressive decrease of feeder cells concentration over several passages.. The dishes were originally seeded with around 2,7 x10⁴ feeder cells/cm², then around 1,8 x 10⁴ feeder cells/cm² between passage 32 and 38, then around 1,4 x10⁴ cells/cm² between passage 39 and 44, then around 1 x 10⁴ feeder cells/cm² between passage 45 and 47, then around 0,7 x 10⁴ feeder cells/cm² between passage 48 and 50, and finally from passage 51 dishes were seeded only with avian cells and without feeder cells,. At the end of the feeder depletion, the dishes are seeded with 9 x 10⁴ avian cells/cm² to 12,7 x 10⁴ avian cells/cm². The depletion of feeder cells started at passage 32 and ended at passage 51. During the depletion of feeder cells, the duck ES cells are seeded in culture dishes at a higher concentration than in step a), about around 9 x 10⁴ cell/cm² to 12,7 x 10⁴ cell/cm². After several passages without feeder cells, growth parameters (Population Doubling Time (PDT) and Density) were studied to confirm cell stability and robustness and to initiate the cell growth as suspension. Cells are considered as enough robust to be submitted to a culture in suspension if, PDT is lower than around 40 hours and cell density higher than around 26x10⁴ cells/cm². Moreover, cells morphology should be: round, refringent, very small and the cells shall not attached to the plastic dish too much.

In the case of the EB26 cell development, culture in suspension were initiated at passage 53.7 x 10⁶ cells were transferred in a Ultra Low attachment dish and maintained under constant agitation at around 50 to 70 rpm. For the next passages, cells were seeded in T175 flasks (Sarsted, ref 831812502) at a concentration comprise between 0,4 to 0,5x10⁶ cells/mL. Following a short period of adaptation to the new conditions of culture, cells PDT decreased from around 160 H to 40 hours. Regarding this good evolution, at passage 59, a new set of deprivation was performed. Thus vitamins, sodium pyruvate, beta-mercaptoethanol and non essential amino acids were removed. Thus after passage 59, the culture medium was supplemented with 5 % FBS, 0,5 X yeastolate and 2,5 mM glutamine only. The serum depletion is performed on cell suspensions already depleted in growth factor, feeder cells, vitamins, non essential amino acids, sodium pyruvate and beta-mercaptoethanol. Serum depletion was performed by a progressive decreasing starting from 5 % serum, then 2.5 %, then 1,5 %,of serum concentration in SFM cell culture medium to finally reach 0 % serum in SFM cell culture medium. Serum depletion started at passage 61 and ended at passage 79. At the end of serum depletion, anchorage independent duck EB26 cells were able to grow at 39°C in absence of grow factors, in absence of feeder cells, in serum free medium. EB26 cells were then adapted to growth in absence of 0.5X yeastolate at 37°C, by decreasing cell culture temperature at passage 80.

After the obtaining of EB26 cells that are able to grow at 37°C in the SFM GTM-3 supplemented by 2,5 mM glutamine, some further adaptation were made by dilution or progressive adaptation on new SFM formulations as Excell 63066, Excell 66444, Excell CHO ACF. The subcloning of suspension duck EB26 cell could also realized in presence or absence of yeastolate.

### EXAMPLE 4: Duck EBx cell line EB24

### 4.1 - RAW MATERIAL

### Duck Eggs, Feeder cells, additives, Buffers and Fixators, Cryopreservative agents,

### Fetal Calf Serum & dissociating agents (Idem as Example 3).

Duck eggs from Peking strains GL30 were used.

### Medium

Medium EX-CELL*^{™}* 65319, 63066 and 66444 (SAFC, customized medium)
Medium GTM-3 (Sigma, Cat n° G9916)
DMEM F12 (Cambrex, Cat n° BE04-687)
DMEM (Cambrex, Cat n° BE 12-614F)

### Factors

Six different recombinant factors were used:
□ Recombinant Human Ciliary Neurotrophic Factor (CNTF) (Peprotech Inc, Cat n° 450-13)
□ Recombinant Human Insulin Like Factor I (IGF1) (Peprotech Inc, Cat n° 100-11)
□ Recombinant Human Interleukin 6 (IL6) (Peprotech Inc, Cat n° 200-06)
□ Recombinant Human soluble Interleukin 6 receptor (sIL6r) (Peprotech Inc, Cat n° 200-06 R)
□ Recombinant Human Stem Cell Factor (SCF) (Peprotech Inc, Cat n° 300-07)
□ Recombinant Human basic Fibroblast Growth Factor (bFGF) (Peprotech Inc, Cat n° 100-18B)

All those factors, excepted IL6r, are produced in E. Coli bacteria. Soluble IL6r is expressed in transfected HEK293 cells.

### 4.2 - PROCESS OF ESTABLISHMENT OF DUCK EBx® CELL LINE EB24

The duck embryos were collected as previously described with EB66. The duck embryos were placed in 50 ml tubes containing PBS 1X.The duck embryos are then mechanically dissociated and seeded on an inactivated layer of feeder STO cells into complete culture medium at 39°C, 7,5 % CO₂. The feeder cells were seeded in 6 well plates or dishes at around 2,7 x10⁴ cell/cm². The complete culture medium is composed of serum free medium DMEM-Ham F12 supplemented with 10 % fetal bovine serum, with IGF1, CNTF, Il-6, Il-6R, SCF and FGF at a final concentration of 1ng/ml, and with 1 % non-essential amino acids, with 1 % of mixture of vitamins of commercial origin, with sodium pyruvate at a final concentration of 0,1 mM, with beta-mercapto-ethanol at a final concentration of 0.5 mM, glutamine at a final concentration of 2,1 mM, penicillin at a final concentration of 100 U/ml, streptomycin at a final concentration of 100 µg/ml and 1X yeastolate. Rapidly after the first passages of the cells, the mixture of antibiotics is no longer added to the medium. The expression rapidly is understood to mean after the first 3 to 9 passages in general.

The duck ES cells are cultured in the DMEM-Ham F12 complete medium up to passage 7. After passage 7, the base medium is modified and DMEM-Ham F12 complete medium is replaced by the GTM-3 complete medium supplemented with 10 % fetal bovine serum, with IGF1, CNTF, Il-6, Il-6R, SCF and FGF at a final concentration of 1ng/ml, with 1 % non-essential amino acids, with 1 % of mixture of vitamins of commercial origin, with sodium pyruvate at a final concentration of 0,1 mM, with beta-mercapto-ethanol at a final concentration of 0.5 mM, glutamine at a final concentration of 2,1 mM, penicillin at a final concentration of 100 U/ml, streptomycin at a final concentration of 100 µg/ml and yeastolate 1X. Thus, at passage 11, the serum concentration is decreased at 5 % and SCF, IL6, IL6r and bFGF are removed for the medium. So, from passage 11, the medium is composed of 5 % FBS, with IGF1 and CNTF at a final concentration of 1ng/mL with 1 % non-essential amino acids, with 1 % of mixture of vitamins of commercial origin, with sodium pyruvate at a final concentration of 0,1 mM, with beta-mercapto-ethanol at a final concentration of 0.5 mM, glutamine at a final concentration of 2,1 mM, penicillin at a final concentration of 100 U/ml, streptomycin at a final concentration of 100 µg/ml and yeastolate 1X. A simultaneous withdrawal of IGF1 and CNTF is performed at passage 22. No recombinant factors are present in the GTM-3 culture medium after passage 22. Duck cells were maintained in a such medium between passage 23 and passage 28. When the duck ES cells from Pekin duck embryos are passaged from a culture dish to another, the seeding of culture dish was performed with around between 7 x 10⁴/cm² to 12 x 10⁴/cm² of duck ES cells in the complete culture medium. Preferably, the seeding is made with around 7.3 x 10⁴/cm² (4 x 10⁶ cells/55cm² or 4 x 10⁶ cells/100 mm dish). Then, after passage 28, depletion of feeder cells is performed by a progressive decrease of feeder cells concentration over several passages. The dishes were originally seeded with around 2,7 x10⁴ feeder cells/cm², then around 1,8 x 10⁴ feeder cells/cm² between passage 29 and 33, then around 1,4 x10⁴ cells/cm² between passage 34 and 37, then around 1 x 10⁴ feeder cells/cm² between passage 38 and 42, then around 0,7 x 10⁴ feeder cells/cm² between passage 43 and 46, and finally from passage 47 dishes were seeded only with avian cells and without feeder cells,. At the end of the feeder depletion, the dishes are seeded with 9 x 10⁴ avian cells/cm² to 12,7 x 10⁴ avian cells/cm². The depletion of feeder cells started at passage 29 and ended at passage 47. During the depletion of feeder cells, the duck ES cells are seeded in culture dishes at a higher concentration than in step a), about around 9 x 10⁴ cell/cm² to 12,7 x 10⁴ cell/cm². After several passages without feeder cells, growth parameters (Population Doubling Time (PDT) and Density) were studied to confirm cell stability and robustness and to initiate the cell growth as suspension. Cells are considered as enough robust to be submitted to a culture in suspension if, PDT is lower than around 40 hours and cell density higher than around 26 x 10⁴ cells/cm². Moreover, cells morphology should be: round, refringent, very small and the cells shall not attached to the plastic dish too much. In the case of the EB24 cell development, culture in suspension is initiated at passage 48. 8 x10⁶ cells were transferred in a Ultra Low attachment dish and maintained under constant agitation at around 50 to 70 rpm. For the next passages, cells were seeded in T175 flasks (Sarsted, ref 831812502) at a concentration comprise between 0,4 to 0,5x10⁶ cells/mL. Following a short period of adaptation to the new conditions of culture, cell PDT decreased from around 248 H to 128 hours and the next step of deprivation is then performed. Thus at passage 52, vitamines, non essential amino acids, sodium pyruvate and beta mercaptethanol are removed. Regarding the good evolution of the PDT reaching 44 hours, at passage 56, from passage 57, the serum deprivation was initiated. Thus from passage 57, the culture medium GTM-3 was supplemented with 5 % FBS, 1X yeastolate and 2,5 mM glutamine only. The serum depletion is performed on cell suspensions already depleted in growth factors, feeder cells, vitamins, non essential amino acids, sodium pyruvate and beta-mercaptoethanol. Serum depletion was performed by a progressive decreasing starting from 5 % serum, then 2.5 %, then 2 %, then 1.5 % of serum concentration in SFM cell culture medium to finally reach 0 % serum in SFM cell culture medium. Serum depletion started at passage 57 and ended at passage 77. During this serum depletion, adaptation to growth at 37°C was also performed. Thus at passage 65, cells growing in the culture medium supplemented with 2,5 % FBS were transferred at 37°C avoiding a progressive temperature shift. At the end of serum depletion, anchorage independent duck EB24 cells were able to grow at 37°C in absence of grow factors, in absence of feeder cells, in serum free medium.

After the obtaining of duck EB24 cells able to grow at 37°C in the SFM GTM-3 supplemented by 2,5 mM glutamine, some further adaptation were made by dilution or progressive adaptation in new SFM formulations as Excell 63066, Excell 66444, Excell CHO ACF. The subcloning of suspension duck EB24 were performed, an duck EB24-12 subclone were selected because of its good performance to efficiently replicate viruses.

### EXAMPLE 5: EXPRESSION VECTORS AND CONSTRUCTS

### 5.1- Parental empty expression vector: pVVS431 (pKNexp)

VIVALIS vector backbone comprises a single resistance cassette to allow plasmid amplification in *E.coli* and clone selection after transfection of expression vectors in avian EBx® cells. The *nptII* gene encodes the neomycin phospotransferase protein and gives to the organism resistance to Kanamycin in prokaryotes and to Neomycin in eukaryotes. Transcription is driven by both prokaryotic and eukaryotic promoters cloned in tandem upstream *nptII* gene. cDNAs encoding proteins of interest is cloned within a MCS (multiple cloning site) located in an empty expression cassette harbouring an easely exangeable promoter (i.e CMV promoter, ...), an intron and a polyA region (Figure N°1).

The following is an example expression vectors bearing RSV promoter sequence; similar procedures were used to construct expression vectors with CMV, EF&/HTLV, SV40, beta-Actin, mPGK, eiF4alpha, CAG, ENS-1 promoters.

### 5.2- Vector bearing RSV promoter driving IgG1 light chain expression : pVVS452 (pRSV-IgG1-L)

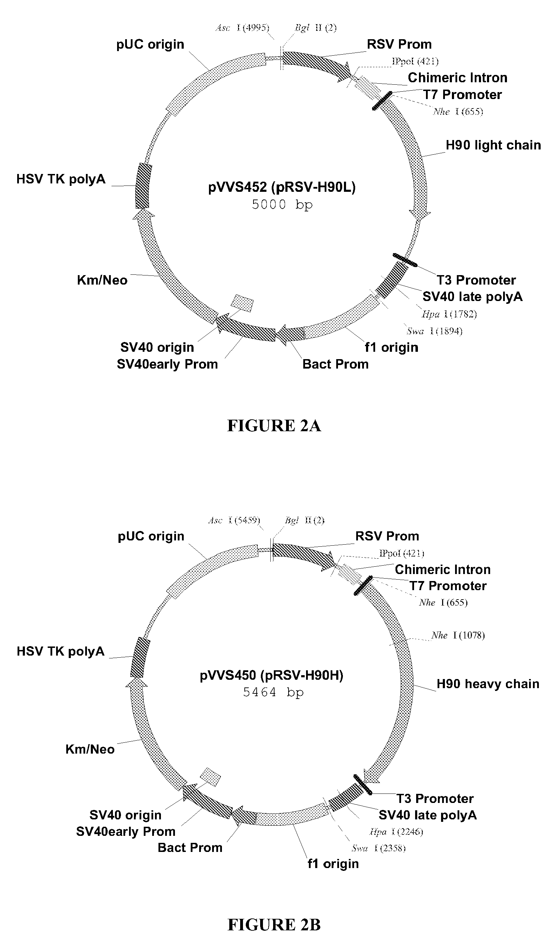

Human-mouse chimeric Mab IgG1 (kappa light chain) was kindly obtained from MAT-Biopharma (Evry, France). The blunted NcoI-KpnI DNA fragment from plgG1-L-clone10 (MAT-Biopharma) was cloned within blunted NheI-NotI sites ofpKNexp MCS region to generate pVVS451 (pCMV-IgG1-L). In pVVS451 Mab light chain expression is driven by a CMV promoter. RSV promoter (Rous Sarcoma virus) was obtained from Philippe Moullier (Inserm U649 - Laboratoire de Thérapie Génique, CHU Hotel-Dieu, Nantes). RSV promoter was PCR amplified from pAAV5RSVLacZ with SO29 (RSV-Bgl2F: ATAAGATCTGCGATGTACGGGCCAGATA) (SEQ ID N° 7) and SO30 (RSV-IPpoIR: TATGCTACCTTAAGAGAGTCCAGCTTGGAGGTGCACACC) (SEQ ID N° 8) primers. RSV promoter PCR fragment and pVVS451 were digested with BgIII and IPpoI. pVVS451 CMV promoterless DNA fragment was agarose gel purified and ligated to RSV promoter in order to create pVVS452 (Figure 2A).

### 5.3- Vector bearing RSV promoter driving IgG1 heavy chain expression: pVVS450 (pRSV-H90H)

Mab gamma1 isotype was obtained from MAT-Biopharma (Evry, France). The blunted NcoI-HindIII DNA fragment from pHIgG1- H-clone28 (MAT-Biopharma) was cloned within blunted NheI-NotI sites of pKNexp MCS region to generate pVVS449 (pCMV-HIgG1L). In pVVS449 Mab heavy chain expression is driven by a CMV promoter. RSV promoter PCR fragment and pVVS449 were digested with BglII and IPpoI. pVVS449 CMV promoterless DNA fragment was agarose gel purified and ligated to RSV promoter in order to create pVVS450 (Figure 2B).

### 5.4- Dual vectors expressing both H90 light and heavy chains under control of RSV promoter: pVVS455 (pRSV-LH-IgG1) and pVVS460 (pRSV-HL-IgG1)

The IgG1 light chain expression cassette driven by the RSV promoter was amplified by PCR from pVVS452 using SO29 and AS455 (AscBamSV40polyAR : ATAGGCGCGCCGGATCCCGATCCTTATCGGATTTTACCAC) primers (SEQ ID N° 9). This DNA fragment bearing the RSV promoter, the H90 light chain and the SV40 late polyA was digested by AscI and BamHI. Recipient pVVS450 vector was AscI-BgIII digested. 5' ends of the linearized vector were dephosphorylated before ligation with the purified amplified DNA fragment to generate pVVS455 (pRSV-LH-IgG1) (Figure 3A).

Same SO29 and AS455 primers were used to amplify from pVVS450 the IgG1 heavy chain expression cassette driven by the RSV promoter. This DNA fragment bearing the RSV promoter, the IgG1 heavy chain and the SV40 late polyA was digested by AscI and BamHI. Recipient pVVS452 vector was AscI-BgIII digested. 5' ends of the linearized vector were dephosphorylated before ligation with the purified PCR DNA fragment to generate pVVS460 (pRSV-HL-IgG1) (Figure 3B).

### 5.5- NR13 expressing vector bearing a puromycin resistance: pVVS438 (pNR13-puro)

RT-PCR experiment was performed on total RNA purified from S86N45 Gallus gallus species to generate the cDNA encoding NR13 anti-apoptotic gene. Oligonucleotides AS376 (Nhe1-NR13F: ACGCTAGCATGCCGGGCTCTCTGAAGGAGGAGAC) (SEQ ID N° 10) and AS377 (Not1-NR13R: GAGCGGCCGCCTACCGCAC CACCAAGAGAAAAGCTAATC) (SEQ ID N° 11) bearing NheI and NotI sites, respectively, were used to generate the NR13 cDNA fragment. A pCiNeo based vector within which the CMV promoter was deleted and replaced by the RSV promoter was used as the recipient of NR13 cDNA. NheI and NotI digestion of both DNA was followed by a ligation experiment. Ligation products were used to run a PCR amplification with primers SO29 and AS4349 to get the NR13 RSV driven promoter expression cassette. This PCR fragment was digested by BglII and SwaI. Recipient pVVS437 vector was BglII-SwaI digested. Both purified insert and vector were ligated together to create pVVS438 (pNR13-puro).

### EXAMPLE 6: TRANSIENT TRANSFECTION IN SERUM FREE MEDIUM AND ISOLATION OF BEST PROMOTERS

### 6.1 - Transient transfection in chicken EBx cells

Transient expression in adherent chicken EBx cells of two reporter genes (red fluorescent protein dsREDnuc and the human blood coagulation factor IX) each driven by 8 different promoters was performed to isolate the best promoter.

The 8 different promoters tested in this experiment were: CMV promoter (IE human cytomegalovirus promoter); SV40 promoter (simian virus 40); human beta-actin promoter; mouse PGK promoter (phosphoglycerate kinase); RSV-LTR (LTR of the Rous Sarcoma virus); Human eIF4alpha promoter (translation initiation factor4 alpha); EF1alpha-HTLV composite promoter (human EF1 elongation factor liked to the 5'UTR region of HTLV1 virus); CAG composite promoter (CMV enhancer linked to the chicken beta-actin promoter).

Expression vectors bearing these 8 promoters (Table 1) were transiently transfected in chicken adherent EBx® cell line using lipofectamine^{™} or fugene^{™} reagents, or by electroporation. The 4 promoters for which the highest transient expression of reporter genes where observed were : EF1alpha-HTLV, RSV, CAG and CMV, and were selected and further used to express chimeric human/mouse IgG1 in EBx® cells.

**Table 1 : list of promoters driving cDNA expression used in transient expression experiments in order to select promoters with high levels of expression in EBx® cells. GFP: fluorescent reporter protein; DsRed2nuc : fluorescent reporter protein; hFIX : human coagulation factor IX; IgG1-LH or HL : Light and heavy chains encoding IgG1 cloned within the same vector and driven by the same notified promoter. LH : light chain is cloned upstream heavy chain; HL : heavy chain is cloned upstream light chain. pVVSxxx correspond to vectors names as registred in Vivalis' database.**

| **Promoter** | **cDNA** | **Vector name** |
|---|---|---|
| **SV40 (simian virus early promoter)** | DsRed2nuc | pVVS405 |
| | hFIX | pVVS415 |
| **Beta-actin (human)** | DsRed2nuc | pVVS418 |
| | hFIX | pVVS421 |
| **mPGK (mouse phosphoglycerate kinase)** | DsRed2nuc | pVVS407 |
| | hFIX | pVVS420 |
| **CMV (Human immediate early promoter from human cytomegalovirus (herpes virus 5))** | GFP | pVVS525 |
| | DsRed2nuc | pVVS410 |
| | hFIX | pVVS413 |
| | IgG1-LH | pVVS488 |
| | IgG1-HL | pVVS489 |
| **LTR-RSV (LTR from Rous Sarcoma virus)** | DsRed2nuc | pVVS408 |
| | hFIX | pVVS417 |
| | IgG1-LH | pVVS455 |
| | IgG1-HL | pVVS460 |
| **eIF4alpha (human translation initiation factor 4)** | DsRed2nuc | pVVS406 |
| | hFIX | pVVS416 |
| | IgG1-LH | pVVS510 |
| | IgG1-HL | pVVS511 |
| **EF1/HTLV (composite promoter : human elongation factor 1+ HTLV 5'UTR)** | DsRed2nuc | pVVS404 |
| | hFIX | pVVS414 |
| | IgG1-LH | pVVS490 |
| | IgG1-HL | pVVS491 |
| **CAG (composite promoter : CMV enhancer + chicken beta-actin promoter)** | DsRed2nuc | pVVS424 |
| | hFIX | pVVS423 |
| | IgG1-LH | pVVS492 |
| | IgG1-HL | pVVS493 |

The promoters that allow to get the highest IgG1 expression in EBx® cells were EF1alpha-HTLV, RSV and CMV (data not shown), and were further used to perform stable transfection experiments to express chimeric human/mouse IgG1 in EBx® cells.

### 6.2 - Transient transfection in duck EBx cells

### Methods

Expression vectors [pEF1/HTLV - IgG1 light and heavy chain] (pvVS490) were transiently transfected by nucleofection (Amaxa, DE) in suspension duck EBx cells in serum free medium (Excell GTM 3 medium from SAFC Biosciences) supplemented with 2.5 mM Glutamine in 6 wells plate. IgG1 expression were monitored by ELISA assay, 24H and 48h hours after transfection . Control nucleofection was performed without DNA.

### Results

Duck EBx cells transiently expressed up to approximatively 4.5 µg/ml of IgG1 antibody (Figure 4).

### EXAMPLE 7: STABLE TRANSFECTION & BEST PRODUCER CLONES IDENTIFICATION

### 7.1 - Materials & Methods:

### Cells:

Chicken EB14 cells in serum free medium (Excell - from SAFC Biosciences) supplemented with 2.5mM glutamine.

### Expression vectors:

pVVS490(pEF1/HTLV) and pVVS455 (pRSV)

### Transfection:

The expression vectors pEF1/HTLV (pVVS490) or pRSV (pVVS455) were transfected into EB14 cells by electroporation (Amaxa). Three days post-transfection, the selection agent (0.25 mg/ml of geneticin) is added to the cell culture medium. The geneticin resistant clones were isolated, picked up and cultured in larger vessels (microplates, flasks, then bioreactors).

### ELISA:

An ELISA screening assay was performed on stably transfected clones to detect antibody expression level in supernatant. This assay employs the quantitative sandwich enzyme immuno assay technique. An anti IgG-Fc specific antibody is pre-coated onto a 96 well-plate. Standards, samples and conjugates are added to the wells and any IgG present is sandwiched by the immobilized antibody and a second enzyme-linked monoclonal antibody specific for IgG-kappa. Following a wash to remove any unbounded substances and/or antibody-enzyme reagent, a substrate solution is added to the wells and coloration develops in proportion to the amount of IgG bound. Reaction is stopped and coloration intensity is measured (O.D. 490 nm). A standard curve is constructed by plotting the mean absorbance for each standard on the y-axis against the concentration on the x-axis and draw a best fit curve through the points of the graph. The concentration of each unknown sample is determined by calculating the concentration of the IgG corresponding to the mean absorbance from the standard curve. For samples, the concentration determined from the standard curve must be multiplied by the dilution factor.

### 7.2 - Results:

Many EBx® cells clones stably transfected with expression vector encoding a heavy and light chains cDNA of chimeric human-mouse IgG1 were obtained and selected by ELISA. The best producer clones were selected and cultured in larger vessels (figure 5). One stably transfected EBx clone (pEF1/HTLV) expressing IgG1 were adapted to the culture in suspension and were further cultured in 3 liter stirred-tank bioreactor. After 9 days of culture, cell density reached 16 million cells/ml and IgG1 concentration in cell culture supernantant was around 0.25 g/l (Figure 6).

### EXAMPLE 8: GLYCOSYLATION PATTERN ANALYIS OF EBx® PRODUCED IgG1 MONOCLONAL ANTIBODY

A capillary elctrophoresis analysis of glycans attached on EBx produced IgG1 were performed an compared to capillary elctrophoresis analysis of glycans attached on human seric IgGs. The results obtained demonstrated that EBx glysosylation profile was similar to human glycosylation profile (Figure 7A).

A comparison of glycosylation pattern of an IgG1 produced in CHO (Chinese Hamster Ovary) and in chicken EB14 cells were also performed using Maldi-Toff mass spectrum analysis of N-linked glycans. Chicken EB14 cells enable production of antibodies population that are less fucosylated than with usual CHO cells (Figure 7B) or HEK cells (data not shown).

The glycosylation profile of an IgG1 produced in one stably transfected clone of chicken EB14 cells were analysed by Maldi-Toff mass spectrum analysis. The structure of N-oligosaccharide attached to the CH2 domain of each IgG1 heavy chain at residue Asn 297 were analyzed (Figure 8). Most of IgG1 antibodies produced in this chicken EB14 clone has a common N-linked oligosaccharide structure of a bi-antennary type that comprises long chains with terminal GlcNac, that are galactosylated. An important part (48%) of IgG1 antibodies population is not fucosylated (Figure 7B and 8).

### EXAMPLE 9: INHIBITION ANALYIS OF TUMOR CELLS PROLIFERATION WITH IgG1 PRODUCED IN EBx® CELLS VS HYBRIDOMA

An assay was developed to measure cellular proliferation inhibition activity of an IgG1 immunoglobulin either produced in chicken EB14 cells or in an hybridoma. The IgG1 antibody is directed against a CD marker expressed on human tumor cells surface.

Tumor cells were grown in presence of tritiated thymidine and were incubated with monocytes or natural killer T cells purified from a normal patient, in presence of IgG1 immunoglobulin. The assay measures amount of tritiated thymidine incorporated in tumor cells as a consequence of IgG1 mediated tumor cell lysis by monocytes or NK cells. After 4 days in culture, low amount of tritiated thymidine were incorporated in tumor cells treated NK or monocytes cells and with IgG1 antibody produced in chicken EB14 cells. In the opposite, a higher level oh H3-thymidine was observed in tumor cells treated with NK or monocytes cells and with IgG1 antibody produced in hybridoma.

Therefore, IgG1 produced in chicken EB14 cells display a better cell mediated cytotoxic activity that the same antibody produced in hybridoma (Figure N° 9).

### EXAMPLE 10: ADCC ACTIVITY OF IgG1 MAB PRODUCED IN EBx® CELLS 10.1 - Methods

PBMCs from two healthy donors were isolated using standard density centrifugation procedures and were suspended at 5 x 10⁶ cells/ml in RPMI cell culture medium.

The disease related target cells were grown by standard tissue culture methods, harvested from the exponential growth phase with a viability higher than 90%, washed in RPMI cell culture medium, labeled with 100 micro-Curies of ⁵¹Cr, washed twice with cell culture medium, and resuspended in cell culture medium at a density of 10⁵cells/ml.

One hundred microliters of disease related target cell suspension were transferred into each well of a 96-well microtiter plate.

Antibody directed to antigenic surface marker of said disease target cells were serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above. The 96-well microtiter plate was then centrifuged at 50 x g for 1 minute and incubated for 1 hour at 4°C.

50 microliters of the PBMC suspension were added to each well to yield an effector: target cell ratio of 25:1 and the plates are placed in an incubator under 5 % CO₂ atmosphere at 37°C for 4 hours.

The cell-free supernatant from each well is harvested and the experimentally released radioactivity (ER) is quantified using a gamma counter.

### 10.2 - Results

Two independent experiments performed using NK cells purified from PBMCs of two distinct healthy donors showed that IgG1 antibody directed against disease related target cells and produced in chicken EB14 cells have an increased ADCC activity compared to the same antibody produce in CHO cells (Figure 10).

### EXAMPLE 11: GROWTH ANALYSIS OF ADHERENT CHICKEN EBX® CELLS STABLY TRANSFECTED WITH NR13 ANTI-APOPTOTIC GENE

Two distinct expression vectors pVVS437 and pVVS438 vectors were constructed. pVVS437 harbors only the puromycine resistance gene. PVVS438 allows the expression of puromycine resistance and of the chicken anti-apoptotic gene NR13. Chicken adherent EBx® cells were transfected with pVVS437 or pVVS438 vectors and best producers clones were selected.

Stably transfected EBx® cells that express NR13 protein were cultured in adherence in 100 mm dishes. EBx® cells that do not expressed NR13 protein do not survive into culture and most of them are dead after 6 to 7 days in culture. Only a small and stable proportion of EBx® expressing NR13 protein are dead in the culture, the majority of NR13 EBx® cells is staying alive for a longer period of time in culture (Figure 11).

## Claims

1. An avian EBx® cell transfected with at least one expression vector comprising at least one expression cassette comprising nucleic acid sequence encoding a recombinant protein of interest operably linked to a promoter sequence capable of effecting expression of said protein in said cell.

2. The EBx® cell according to claim 1 wherein the expression vector further comprises at least one expression cassette comprising at least a nucleic acid sequence encoding a selectable marker operably linked to a promoter sequence capable of effecting expression of said selectable marker in the host cell.

3. The EBx® cell according to claims 1 to 2 wherein the selectable marker is selected from glutamine synthase, xanthine guanine phosphoribosyl transferase, puromycin, hygromycin B, neomycin gene and dihydrofolate reductase (DHFR).

4. The EBx® cell according to claim 3 wherein the promoter sequence is selected from a cytomegalovirus (CMV) promoter, a simian virus 40 (SV40) early promoter, a Herpes Simplex virus (HSV) thymidine kinase promoter, a rous-sarcoma virus (RSV) promoter, a murine Phospho-glycerate kinase promoter, an eIF4alpha promoter, a chimeric EF1alpha/HTLV promoter, a CAG promoter, a beta-actin promoter.

5. The EBx® cell according to claims 1 and 4 wherein said expression cassette(s) further comprises one or more control element or regulatory sequences selected in the group consisting of transcriptional initiation sequence, enhancer sequence, intronic sequence, polyadenylation sequence, termination sequence, chromatin insulator element.

6. The EBx® cell according to claim 5 wherein the chromatin insulator element is selected from Boundary Elements (BE), Matrix Attachment Region (MAR), Locus Control Region (LCR), Universal Chromatin Opening Elements (UCOE).

7. The EBx® cell according to claims 1 to 6 wherein said expression vector comprises at least:
- a first expression cassette comprising the following DNA sequences in the following order: CMV promoter sequence of SEQ ID N°1, intronic sequence, cDNA sequence encoding a recombinant protein of interest, polyadenylation sequence; and
- a second expression cassette comprising the following DNA sequences in the following order: SV40 promoter, neomycin gene, polyadenylation sequence.

8. The EBx® cell according to claims 1 to 6 wherein said expression vector comprises at least:
- a first expression cassette comprising the following DNA sequences in the following order: chimeric EF1alpha/HTLV promoter sequence of SEQ ID N° 2, intronic sequence, cDNA sequence encoding a recombinant protein of interest, polyadenylation sequence; and
- a second expression cassette comprising the following DNA sequences in the following order: SV40 promoter, neomycin gene, polyadenylation sequence.

9. The EBx® cell according to claims 1 to 6 wherein said expression vector comprises at least in the following order :
- a first expression cassette comprising the following DNA sequences in the following order: CMV promoter of SEQ ID N°1, intronic sequence, cDNA sequence encoding the heavy chain of an antibody or a fragment thereof, polyadenylation sequence;
- a second expression cassette comprising the following DNA sequences in the following order: CMV promoter of SEQ ID N°1, intronic sequence, cDNA sequence encoding the light chain of the antibody or a fragment thereof, polyadenylation sequence;
- a third expression cassette comprising the following DNA sequences in the following order: SV40 promoter, neomycin gene, polyadenylation sequence.

10. The EBx® cell according to claims 1 to 6 wherein said expression vector comprises at least in the following order:
- a first expression cassette comprising the following DNA sequences in the following order: chimeric EF1alpha/HTLV promoter of SEQ ID N° 2, intronic sequence, cDNA sequence encoding the heavy chain of an antibody or a fragment thereof, polyadenylation sequence;
- a second expression cassette comprising the following DNA sequences in the following order: chimeric EF1alpha/HTLV promote of SEQ ID N° 2, intronic sequence, cDNA sequence encoding the light chain of the antibody or a fragment thereof, polyadenylation sequence;
- a third expression cassette comprising the following DNA sequences in the following order: SV40 promoter, neomycin gene, polyadenylation sequence.

11. The EBx® cell according to claims 1 to 10 wherein the cell further comprises an expression vector comprising at least an expression cassette comprising DNA sequence encoding an anti-apoptotic protein operably linked to a promoter sequence capable of effecting expression of said anti-apoptotic protein in the cell.

12. The EBx® cell according to claim 11 wherein the anti-apoptotic protein is chicken NR13 protein.

13. The EBx® cell according to claims 1 to 12 wherein expression vector(s) is (are) stably incorporated into the chromosomal DNA of the cell.

14. The EBx® cell according to claims 1 to 13 wherein the cell is chicken EBx® cell selected among EB14 and EBv13 cells and duck EBx cells selected among EB24, EB24-12, EB26 and EB66.

15. The EBx® cell according to claim 14 wherein said cell is suspension EBx® cell adapted to serum-free medium.

16. The EBx® cell according to claim 14 wherein cell is adherent EBx® cell adapted to serum-free medium.

17. A method for producing at least one biological product of interest in avian EBx® cell, said method comprising the steps of:
a) preparing EBx® cell according to claim 1 to 16 by transfection with at least one expression vector;
b) culturing said EBx® cell under suitable conditions and in suitable medium; and
c) harvesting the biological product of interest from the EBx® cell, the suitable medium, or both said EBx® cell and said medium.

18. The method according to claim 17, wherein the EBx® cells are transfected by electroporation with at least one expression vector in adherent culture in a serum free medium.

19. The method according to claim 17, wherein the EBx® cells are transfected by liposome-mediated transfection with at least one expression vector in suspension culture in a serum-free medium.

20. The method of claim 17 to 19 wherein the biological product of interest is an antibody molecule, an antibody fragment or a fusion protein that includes a region equivalent to the Fc region of an immunoglobulin, wherein said biological product of interest is having an increased Fc-mediated cellular toxicity.

21. A biological product of interest having chicken or duck EBx® glycosylation.

22. An antibody population produced in EBx® cells and having EBx® glycosylation wherein said antibody population, or fragment thereof, comprises Fc region comprising a large proportion of antibodies carrying a common N-linked fucosylated-oligosaccharide structure of a biantennary-type that comprises long chains with terminal GlcNac that are highly galactosylated, and which confer strong ADCC activity to said antibodies.

23. The antibody according to claims 22 of IgG1 or IgG4 subtypes, produced in EBx® cells, and having an increased ADCC activity compared to the same antibody produced in hybridoma or CHO cells.

24. The biological product of interest according to claim 21 as a medicament.

25. The antibody, or a fragment thereof, according to claims 22 and 23 as a medicament.

26. The use of a biological product according to claim 24, or an antibody according to claim 25, or a fragment thereof, for the preparation of a pharmaceutical composition for the prevention or the treatment of human and animal diseases.

27. A pharmaceutical composition comprising the biological product of claim 24, or an antibody according to claim 25, or a fragment thereof, and a pharmaceutical acceptable carrier.


SEQUENCE LISTING

<110> VIVALIS

<120> Recombinant protein production in avian EBx® cells.

<130> D25555

<160> 11

<170> PatentIn version 3.3

<210> 1
<211> 489
<212> DNA
<213> Human Cytomegalovirus

<220>
<223> CMV promoter sequence

<400> 1
ggcgaccgcc cagcgacccc cgcccgttga cgtcaatagt gacgtatgtt cccatagtaa 60
cgccaatagg gactttccat tgacgtcaat gggtggagta tttacggtaa actgcccact 120
tggcagtaca tcaagtgtat catatgccaa gtccgccccc tattgacgtc aatgacggta 180
aatggcccgc ctagcattat gcccagtaca tgaccttacg ggagtttcct acttggcagt 240
acatctacgt attagtcatc gctattacca tggtgatgcg gttttggcag tacaccaatg 300
ggcgtggata gcggtttgac tcacggggat ttccaagtct ccaccccatt gacgtcaatg 360
ggagtttgtt ttggcaccaa aatcaacggg actttccaaa atgtcgtaat aaccccgccc 420
cgtgacccaa atgggcggta ggcgtgtacg gtgggaggtc tatatagcag agctcgttta 480
gtgaaccgt 489

<210> 2
<211> 538
<212> DNA
<213> Artificial sequence

<220>
<223> EF1/HTLV promoter sequence

<400> 2
agatctgctc cggtgcccgt cagtgggcag agcgcacatc gcccacagtc cccgagaagt 60
tggggggagg ggtcggcaat tgaaccggtg cctagagaag gtggcgcggg gtaaactggg 120
aaagtgatgt cgtgtactgg ctccgccttt ttcccgaggg tgggggagaa ccgtatataa 180
gtgcagtagt cgccgtgaac gttctttttc gcaacgggtt tgccgccaga acacagctga 240
agcttcgagg ggctcgcatc tctccttcac gcgcccgccg ccctacctga ggccgccatc 300
cacgccggtt gagtcgcgtt ctgccgcctc ccgcctgtgg tgcctcctga actgcgtccg 360
ccgtctaggt aagtttaaag ctcaggtcga gaccgggcct ttgtccggcg ctcccttgga 420
gcctacctag actcagccgg ctctccacgc tttgcctgac cctgcttgct caactctacg 480
tctttgtttc gttttctgtt ctgcgccgtt acagatccaa gctgtgaccg gcgcctac 538

<210> 3
<211> 404
<212> DNA
<213> Rous sarcoma virus

<220>
<223> RSV promoter sequence

<400> 3

gcgatgtacg ggccagatat acgcgtatct gaggggacta gggtgtgttt aggcgaaaag 60
cggggcttcg gttgtacgcg gttaggagtc ccctcaggat atagtagttt cgcttttgca 120
tagggagggg gaaatgtagt cttatgcaat actcttgtag tcttgcaaca tggtaacgat 180
gagttagcaa catgccttac aaggagagaa aaagcaccgt gcatgccgat tggtggaagt 240
aaggtggtac gatcgtgcct tattaggaag gcaacagacg ggtctgacat ggattggacg 300
aaccactgaa ttccgcattg cagagatatt gtatttaagt gcctagctcg atacaataaa 360
cgccatttga ccattcacca cattggtgtg cacctccaag ctgg 404

<210> 4
<211> 133
<212> DNA
<213> Artificial sequence

<220>
<223> Chimeric intron sequence

<400> 4
gtaagtatca aggttacaag acaggtttaa ggagaccaat agaaactggg cttgtcgaga 60
cagagaagac tcttgcgttt ctgataggca cctattggtc ttactgacat ccactttgcc 120
tttctctcca cag 133

<210> 5
<211> 222
<212> DNA
<213> Simian virus 40 (SV40)

<220>
<223> PolyA sequence

<400> 5
cagacatgat aagatacatt gatgagtttg gacaaaccac aactagaatg cagtgaaaaa 60
aatgctttat ttgtgaaatt tgtgatgcta ttgctttatt tgtaaccatt ataagctgca 120
ataaacaagt taacaacaac aattgcattc attttatgtt tcaggttcag ggggagatgt 180
gggaggtttt ttaaagcaag taaaacctct acaaatgtgg ta 222

<210> 6
<211> 548
<212> DNA
<213> Gallus gallus

<220>
<223> NR13 chicken sequence

<400> 6
gctagcatgc cgggctctct gaaggaggag acggcgctgc tgctggagga ctacttccag 60
caccgcgccg gcggcgccgc gctgcctccc agcgccacgg cggccgagct gcggcgggcg 120
gcggccgagc tggagcgccg agagcggccc ttcttccgct cctgcgcgcc gctggcgcgg 180
gccgagccgc gggaggcggc ggcgctgctg cggaaggtgg cggcgcagct ggaggccgaa 240
ggcggcctca actggggccg gctgctggcg ctcgtggtgt tcaccggcac gctggcggcg 300
gcgctggccg agagcggctg cgaggaaggg ccgagccgcc tggccgccgc gctggccgcg 360
tacctggccg aggagcaggg cgagtggctg gaggagcacg gcggatggga cggcttctgt 420
cgcttcttcg gcagacatgg ctcccaacca gctgaccaga acagtacctt aagcaatgcc 480
atcatggcag cggcagggtt tggaatagca ggattagctt ttctcttggt ggtgcggtag 540
gcggccgc 548

<210> 7
<211> 28

<212> DNA
<213> Artificial sequence

<220>
<223> RSV-Bgl2F

<400> 7
ataagatctg cgatgtacgg gccagata                                          28


<210> 8
<211> 39
<212> DNA
<213> Artificial sequence

<220>
<223> RSV-IPpolR

<400> 8
tatgctacct taagagagtc cagcttggag gtgcacacc                              39


<210> 9
<211> 40
<212> DNA
<213> Artificial sequence

<220>
<223> AscBamSV40polyAR

<400> 9
ataggcgcgc cggatcccga tccttatcgg attttaccac                             40


<210> 10
<211> 34
<212> DNA
<213> Gallus gallus

<220>
<223> AS376

<400> 10
acgctagcat gccgggctct ctgaaggagg agac                                   34


<210> 11
<211> 39
<212> DNA
<213> Gallus gallus

<220>
<223> AS377

<400> 11
gagcggccgc ctaccgcacc accaagagaa aagctaatc                              39